# EUROPEAN PATENT APPLICATION

(11) **EP 1 598 363 A1**
(43) Date of publication of application: **23.11.2005**
(21) Application number: 05008653.7
(22) Date of filing: 20.04.2005
(51) Int. Cl.: C07K 14/47, C12N 15/12, C07K 16/18

(54) **Protein regulating the concentration of calcium in endoplasmic reticulum**

(30) Priority: 20.04.2004 JP 2004124443
(71) Applicant: The University of Tokyo, Bunkyo-Ku, Tokyo (JP)
(72) Inventor: Mikoshiba, Katsuhiko, Mitaka City Tokyo (JP); Hattori, Mitsuhiro, Kawasaki City Kanagawa Pref. (JP); Higo, Takayasu, Tokyo (JP)
(74) Representative: Wibbelmann, Jobst

(57) **Abstract**

A molecular of controlling a calcium according to a first aspect of the invention is (a) an amino acid sequence shown in an amino acid sequence number 1-406 of a sequence No.1 of a sequence table, or (b) an amino acid sequence wherein a part of the amino acid sequence of the amino acid sequence number 1-406 is deleted, substituted or added.

## Description

### BACKGROUND OF THE INVENTION

### 1. Field of the Invention

This invention relates to a controlling molecular including a specific polypeptide, a recombinant vector including an nucleic acid encoding polypeptide, and an antibody of the polypeptide, and especially relates to a controlling molecular including a specific polypeptide, a recombinant vector including an nucleic acid encoding said polypeptide, and an antibody of the polypeptide, concerning the endoplasmic reticulum.

### 2. Description of Related Art

Ca²⁺ plays a role in regulating a lot of physiology as a second messenger in cells. Ca²⁺ release from endoplasmic reticulum (ER) induced by a stimulation of a cells is mainly induced by actiration of inositol 1, 4, 5 trisphosphate acid receptor (IP₃Rs). IP₃Rs have three types of subtype, and it is thought that there activity is strongly affected on a micro environment of ER, but details of this are unclear.

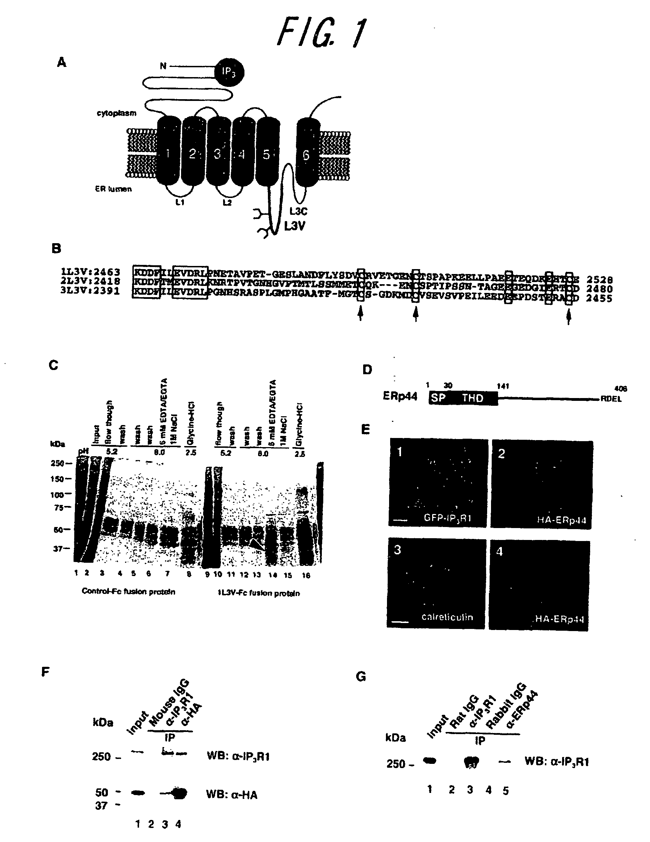

Cytosolic Ca²⁺ concentration ([Ca²⁺]c) is the focal point of many signal transduction pathways and regulates versatile cellular activities ranging from fertilization to cell death (Berridge et al). [Ca²⁺]c is tightly regulated in time, in space and in amplitude because cells extract specific information from these parameters. [Ca²⁺] ions can be supplied to the cytosol from the extracellular space or from intracellular Ca²⁺ stores such as endoplasmic reticulum (ER). Dysfunciton of molecules involved in [Ca²⁺]c regulation, especially those on the ER, is assumed to play major roles in apoptosis (Orrenius et al., 2003) and neuropathological conditions, including Huntington's and Alzheimer's diseases (Mattson and Chan, 2003; Paschen, 2003). IP₃Rs are Ca²⁺ release channels on the ER that play critical roles in generation of complex patterning of [Ca²⁺]c, such as Ca²⁺ waves and oscillations. There are three subtypes in IP₃Rs (IP₃R1, IP₃R2 and IP₃R3) in birds and mammals (Sorrentino et al. 2000). IP₃R1 (Furuichi et al., 1989) is the dominant subtype in brain (Taylor et al., 2002) and has been implicated in neuronal development (tTakei et al., 1998; Xiang et al., 2002), in higher functions of central nervous system (Matumoto et al., 1996; Nishiyama et al., 2002), and in the human neuropathology (Tang et al., 2003). A striking feature of IP₃Rs is the presence of very large cytosolic regions including IP₃ binding region, which contain approximately 80% of the whole structure (Patel et al., 1999). The channel domain contains six transmembrane domains and thus there are three loops that reside in the ER lumen. Among these, the third lumenal loop (L3) is the largest one and it can be divided into two subdomains. The first half (L3V domain) has quite divergent primary sequence among subtypes (Figure 1b), and second half (L3C domain), including pore-forming region is almost completely conserved among types and species. Interestingly, the primary sequence of L3V domain of each subtype is well conserved among species, suggesting that this domain is involved in subtype-specific regulation. However, the function of the L3V domain has been largely unexplored, except that it contains glycosylation sites and can bind Ca²⁺(Sienaert et al)

Recently, it is becoming evident that the intralumenal environment of the ER regulates numerous protein's function (Berridge, 2002).

However, although much effort has been contributed, how it regulates the activity of IP₃Rs remains unclear. Whether or not IP₃R is directly regulated by Ca²⁺ concentration of the ER lumen ([Ca²⁺]) is still a matter of debate (Caroppo et al., 2003). Calreticulin (CRT), and ER lumenal lectin with high Ca²⁺-binding capacity, regulates IP₃-induced Ca²⁺-release (IICR)(Camacho and Lechleiter, 1995; Roderick et al., 1998), but it appears that this is rather indirect effect because CRT does not interact with IP₃Rs (Joseph et al), and CRT critically regulates the activity of sarcoendoplasmic reticulum Ca²⁺ ATPase (SERCA) 2b. In secretory granules, chromogranins associate with the L3C domain of IP₃Rs (Yoo, 2000). Although in vitro studies indicate that chromogranins modulate the activity of IP₃Rs, their role has not been characterized at the cellulae level.

### SUMMARY OF THE INVENTION

It is, therefore, an object of the invention to provide a molecular which regulates calcium dynamic, and mechanism of a regulation of ER calcium concentration.

In order to achieve the above object, the inventors have made various studies with respect to a mechanism of a regulation of ER calcium ER, and found regulatory molecular including a polypeptide of the present invention, an antibody of said polypeptide and a recombinant vector including a nucleic acid encoding said polypeptide.

A molecular of controlling calcium according to a first aspect of the invention is (a) an amino acid sequence shown in an amino acid sequence number 1-406 of a sequence No.1 of a sequence table, or (b) an amino acid sequence wherein a part of the amino acid sequence of the amino acid sequence number 1-406 is deleted, substituted or added.

Furthermore, a expression vector according to a second aspect of the invention comprises (a) a nucleic acid comprising a base sequence shown in a base sequence number 1-1221 of a sequence No.2 of a sequence table, or (b) a nucleic acid wherein a part of the base sequence of the base sequence number 1-1221 is deleted, substituted or added, and the nucleic acid has a homology of 80% for the base sequence.

A molecular of controlling a calcium according to the invention is (a) an amino acid sequence shown in an amino acid sequence number 1-406 of a sequence No.3 of a sequence table, or (b) an amino acid sequence wherein a part of the amino acid sequence of the amino acid sequence number 1-406 is deleted, substituted or added.

Furthermore, a recombinant vector according to the invention comprises (a) a nucleic acid comprising a base sequence shown in a base sequence number 1-1221 of a sequence No.4 of a sequence table, or (b) a nucleic acid wherein a part of the base sequence of the base sequence number 1-1221 is deleted, substituted or added, and the nucleic acid has a homology of 80% for the base sequence.

An antibody of the present invention is against the polypeptide described in claims 1 or 2.

A cultured cell according to the present invention is transformed by said recombinant vector described in claims 2-4.

In a preferable embodiment, the cultured cell according to the invention is characterized that an ability of Ca²⁺ release is reduced by over-expressing the polypeptide described in claims 1 or 3.

In a preferable embodiment, the cultured cell according to the invention is characterized that an ability of Ca²⁺ release is increased by restraining the express of the polypeptide described in claims 1 or 3.

In a preferable embodiment, the cultured cell according to the invention is characterized that an ability of Ca²⁺ release is increased by introducing the antibody described in claim 5 into said cultured cell and inducing an antigen-antibody reaction between the antibody and the polypeptide of claims 1 or 3.

### BRIEF DESCRIPTION OF THE DRAWINGS

The invention will be described with reference to the accompanying drawings, wherein:
FIG. 1 shows an identification of ERp44 as a Binding Protein to the L3V Domain of IP₃R1 (A) Schematic drawing of IP₃Rs contain six membrane-spanning regions (green) and three^lumenal domains, L1, L2 and L3. L3 is divided into L3V (red)and L3C. IP₃ binds to the N-terminal cytosolic region. The L3V contains two glycosylation sites. (B) Sequence comparison of L3V among three subtypes of mouse IP₃Rs. Conserved amino acid residues are shown (red box). Conserved cysteine residues are indicated by arrows. (C) Purification of 1L3V-interacting proteins. Mice cerebellum microsomal fractions solubilized in acidic solution (pH 5.2) were added either to control-Fc or to 1L3V-Fc column. Subsequently, the columns were washed with the same buffer (lanes 3 and 11), neutral buffer (lanes 4-5 and 12-13), and then eluted with neutral buffer with EGTA/EDTA (lanes 6 and 14), the buffer containing 1M NaCl (lanes 7 and 15), and glycine-HCl (lanes 8 and 16). The arrowhead indicates the 45kDa band that specifically binds to 1L3V-Fe, which turned out to be ERp44. (D) Structure of ERp44. Signal peptide (SP, aa 1-30), thioredoxin homology domain (THD, aa 31-141), and ER retention signal RDEL motif, are shown. (E) ERp44 colocalizes with IP₃R1 in the ER. COS-7 cells expressing HA-ERp44 and GFP-IP₃R1 were stained with α-HA (panels 1 and 2). In panels 3 and 4, the cells expressing HA-ERp44 were stained with. Scale bar, 10 µm. (F) In vivo interaction between ERp44 and IP₃R1. COS-7 cells expressing HA-ERp44 and IP₃R1 were treated with a membrane-permeable crosslinker, DSP, and then solubilized. The cell lysates were subjected to immunoprecipitaion with α- IP₃R1 (lane 3), α-HA (lane 4), or control mouse-IgG (lane 2). The lysates (input, lane 1) and immunoprecipitates were analyzed by western blotting with α- IP₃R1 (top) and α-HA (bottom). (G) In vivo interaction between endogenous ERp44 and IP₃R1. The lysates of HeLa cells treated with DSP as (F) were subjected to immunoprecipitation with α- IP₃R1 (lane 3), α-ERp44 (lane 5), rat-IgG (control for IP₃R1, lane 2), or rabbit-IgG (control for ERp44, lane 4). The lysate (input, lane 1) and immunoprecipitates were analyzed by western blotting.

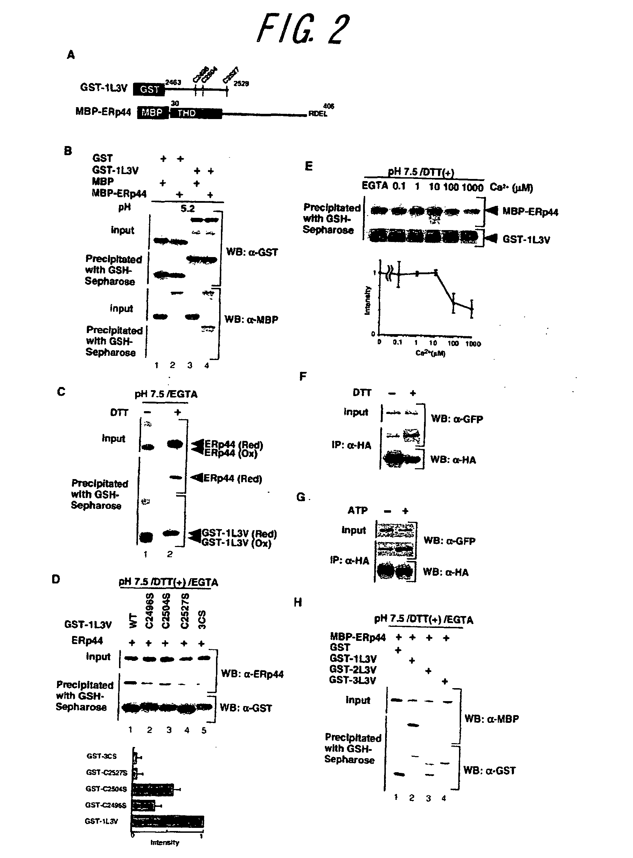

FIG. 2 shows ERp44 specifically interacts with IP₃R1 in a condition-dependent manner. (A) It shows a schematic representation of recombinant proteins. (B) It shows that ERp44 directly interacts with 1L3V in acidic buffer. Purified GST (lanes 1 and 2) or GST-1L3V (lanes 3 and 4) was incubated with purified MBP (lanes I and 3) or MBP-ERp44 (lanes 2 and 4) in acidic solution (pH5.2) containing Ca²⁺(4mM) and then precipitated with GSH-Sepharose beads. Precipitated proteins were subjected to western blotting with α- MBP or α-GST. (C) ERp44 directly interacts with 1L3V in the presence of DTT and EGTA in neutral buffer. To obtain untagged ERp44, purified GST-ERp44 was cleaved by thrombin and then the released GST was removed by the incubation with GSH-Sepharose. This ERp44 protein was incubated with GST-1I3V in neutral solution (pH 7.5) containing 5 mM EGTA in the presence or absence of 3mM DTT. The inputs and bead-bound proteins ware resolved with SDS-PAGE under non-reducing condition and analyzed by western blotting with α- ERp44 (top and middle) or α-GST (bottom). (D) Purified GST-1L3V (lane 1), or its cysteine mutant, C2496S (lane 2), C2504S (lane 3), C2527S (lane 4), or 3CS (C2496S/C2504S/C2527S, lane 5), was incubated with ERp44 (as prepared in C) in the presence of 3mM DTT and 5mM EGTA. Proteins ware then precipitated with GSH-Sepharose and subjected to western blotting with α- ERp44 (middle) or α-GST (bottom). The amount of ERp44 added to the binding reaction is shown on top. Histogram depicts densitometric analysis from three independent experiments. (E) Ca²⁺-dependecy of interaction between 1L3V with ERp44. Binding assay was performed in the presence of EGTA or at the indicated Ca²⁺ concentration, with 3mM DTT. Proteins bound to GSH-Sepharose beads ware then precipitated and subjected to western blotting with α-MBP (top) or α-GST (bottom). Quantitative analysis from three independent experiments. (F) In vivo interaction between IP₃R1 and ERp44 was enhanced under reducing condition. COS-7 cells expressing GFP-IP₃R1 and HA-ERp44 were treated with (+) or without (-) 5mM DTT for 30 min, and then treated with DSP. From the cell lysates, HA-ERp44 was immunoprecipitated. The lysates and immunoprecipitates were analyzed by western blotting with α-GFP (top and middle) or α-HA (bottom). (F) In vivo interaction between IP₃R1 and ERp44 was enhanced after ER Ca²⁺-depletion. COS-7 cells expressing GFP-IP₃R1 and HA-ERp44 were exposed (+) or not (-) to 10µM ATP for 5min, and then treated with DSP. From the cell lysates, HA-ERp44 was immunoprecipitated. The lysates and immunoprecipitates were analyzed by western blotting with α-GFP (top and middle) or α-HA (bottom). (H) ERp44 specifically interacts with IP₃R1, but not with IP₃R2 or IP₃R3. Purified GST (lane 1), GST-1L3V (lane 2), GST-2L3V (lane 3), or GST-3L3V (lane 4) was incubated with MBP-ERp44 in neutral solution (pH7.5) containing EGTA and DTT. Bead bound proteins were subjected to western blotting with α-MBP (middle) or α-GST (bottom).

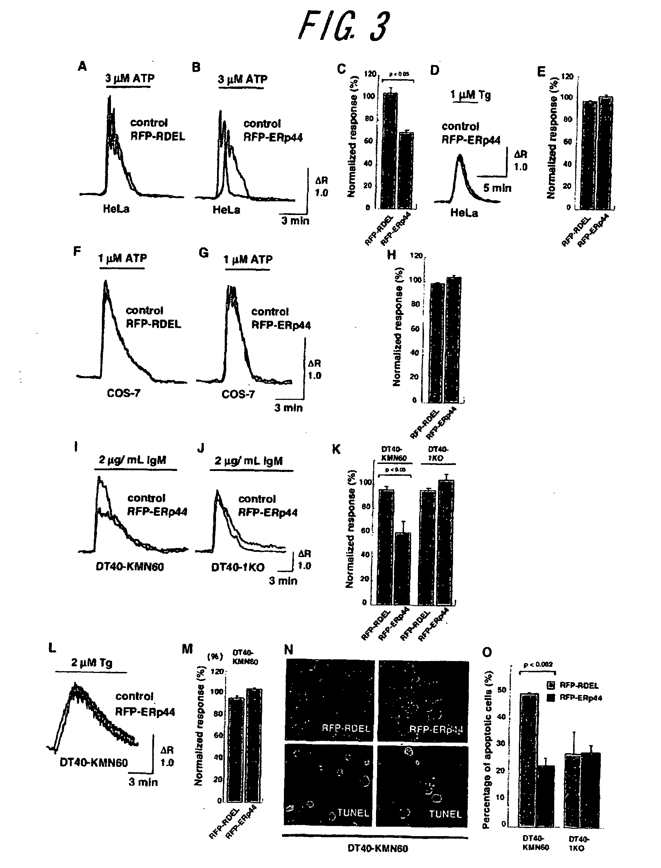

FIG. 3 shows Expression of ERp44 Inhibits IICR in Hela and DT40-KMN 60 Cells, but not in COS-7 or DT40-1KO cells. (A and B) ERp44 inhibits IICR in Hela cells. Cells transfected with either RFP-RDEL (A) or RFP-ERp44 (B) were stimulated with 3µM ATP in nominally Ca²⁺ free midium. Representative Ca²⁺ responses in non-transfected (black trace) and transfected cells (red trace) are shown. (C) Quantitation of results shown in A and B. Normalized responses were calculated with the averaged peak amplitufe of non-transfected cells as 100% (means±SEM). P<0.05 compared to REP-RDEL (Student's t test). (D) The Ca²⁺ storage capacity is unchanged in HeLa cells expressing RFP-ERp44. Cells were stimulated with 1µM Tg and representative Ca²⁺ responses in non-transfected (black trace) and transfected cells (red trace) are shown. (E) Quantitation of the results in D. (F-H) ERp44 does not affect IICR in COS-7 cells. Cells transfected with either RFP-RDEL (F) or RFP-ERp44 (G) were stimulated with 1µM ATP in nominally Ca²⁺ free medium. Representative Ca²⁺ responses in non-trandfected (black trace) and transfected cells (red trace) are shown. (H) Quantitation of results shown in F and G. (l-K) ERp44 inhibits IICR in DT40-KMN60 but not in DT40-1KO cells. Cells were stimulated with 2 µg/mL anti-IgM. Representative Ca²⁺ responses in non-transfected (black trace) and transfected cells (red trace) are shown, and its quantitation is shown in K. (L and M) ERp44 dose not affect Ca²⁺ storage capacity in DT40-KMN60 cells. Ca²⁺ responses in non-transfected (black trace) and transfected cells (red trace), and its quantitation (M) are shown. (N and O) ERp44 inhibits BCR-induced apoptosis in DT40-KMN60 cells, but not in DT40-1KO cells. DT40-KMN60 and DT40-1KO cells expressing RFP-RDEL or RFP-ERp44 were treated with anti-IgM (2 µg/mL) for 24 hr and apoptosis was assessed by TUNEL assay (Roche). At least 250 cells were counted from ten randomly selected fields on each slide. Data are means±SD of percentage TUNEL-positive cells out of all cells expressing RFP-RDEL (gray) or RFP-ERp44 (red) from two independent experiments. P<0.005 compared with RFP-RDEL (Student' t test.

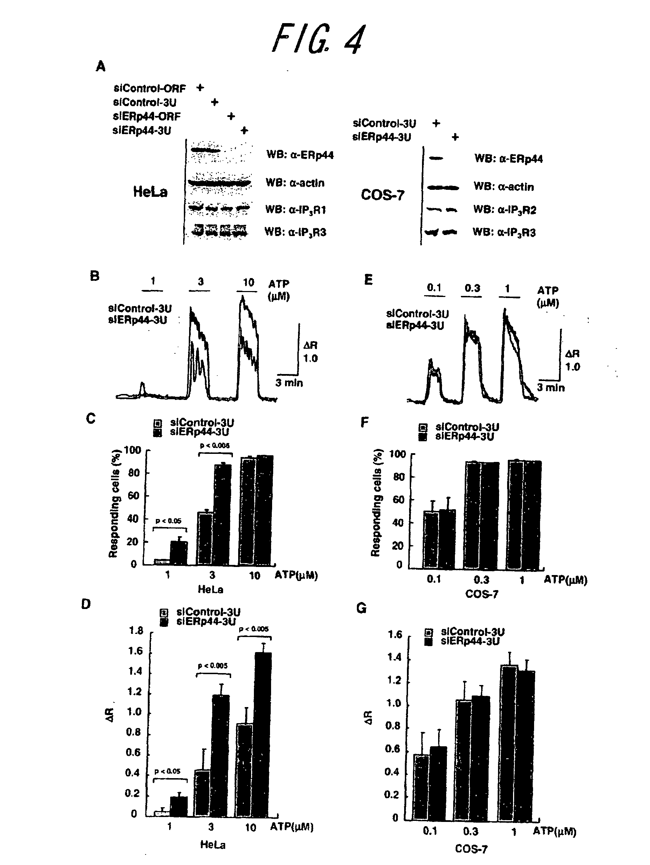

FIG. 4 shows specific Knockdown of ERp44 augments IICR in HeLa cells, but not in COS-7 cells. (A) Knockdown of ERp44 in HeLa and COS-7 cells. The lysate of HeLa (left) and COS-7 cells (right) transfected siRNA was analyzed by western blotting with the indicated antibodies. (B) Knockdown of ERp44 augmented IICR in HeLa cells. Cells were stimulated with 1, 3 and 10 µM ATP in the presence of extracellular Ca²⁺. Representative Ca²⁺ responses in siControl-3U-transfected (black trace) and siERp44-3U-transfected cells (red trace) are shown. (C) Percentages of HeLa cells, transfected either with siControl-3U (gray bars) or with siERp44-3U (red bars), that showed discernible Ca²⁺ responses at the indicated ATP concentrations. Data were calculated from three independent experiments, where at least 150 cells were analyzed. (D) Averages of the peak amplitude of Ca²⁺ response in HeLa cells transfected with siControl-3U (gray columns) or siERp44-3U (red column) from three independent experiments, where at least 150 cells were analyzed, respectively. (E) COS-7 cells were stimulated with 0.1, 0.3 and 1 µM ATP in the presence of extracellular Ca²⁺. Representative Ca²⁺ responses in siControl-3U-transfected (black)trace) and siERp44-3U-transfected cells (red trace) are shown. (F) Percentages of COS-7 cells, transfected either with siControl-3U (gray bars) or with siERp44-3U (red bars), that showed discernible at the indicated ATP concentrations. Data were calculated from three independent experiments. (G) Averages of the peak amplitude of Ca²⁺ response in COS-7 cells transfected with siControl-3U (gray columns) or siERp44-3U (red columns) from three independent experiments, where at least 120 cells were analyzed. All data are means±SD.

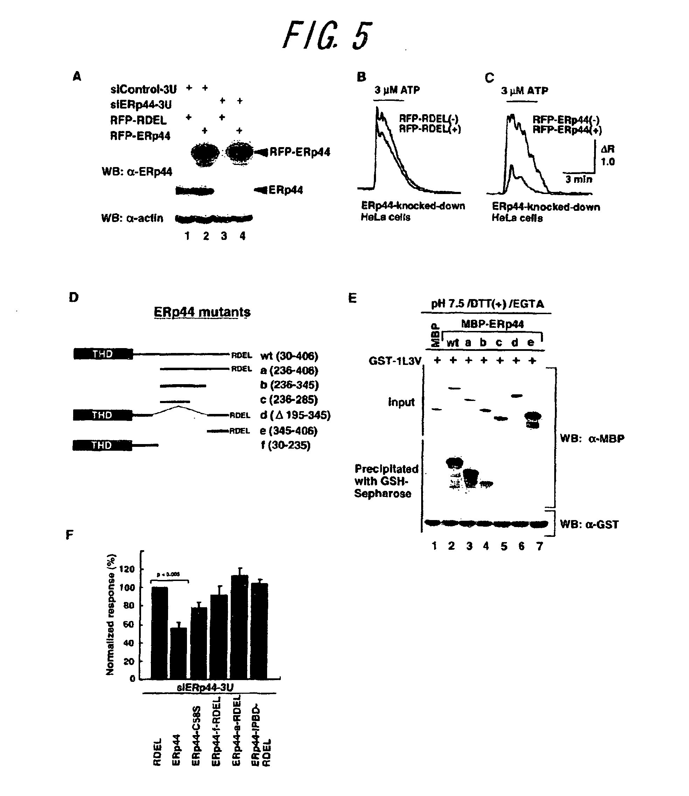

FIG. 5 shows both THD and IPBD of ERp44 are required for inhibition of IICR. (A) Restoration of ERp44 expression in ERp44-knocked-douwn cells. HeLa cells were transfected with siControl-3U. Twenty-four hours later, the cells were transfected with expression vectors for RFP-RDEL or RFP-ERp44. After further 24 hours, cells were harvested and analyzed by western blotting. (B and C) Restoration of ERp44 inhibits IICR. HeLa cells in which endogenous ERp44 had been knocked down were transfected with expression vector for RFP-RDEL (B) or RFP-ERp44 (C). Cells are stimulated with 3 µM ATP in nominally Ca²⁺ free medium. Representative Ca²⁺ responses in non-transfected (black trace) and transfected cells (red trace) are shown. (D) Schematic representation of ERp44 and its mutants. (E) Amino acid residue 235-345 of ERp44 is responsible for interaction with 1L3V. GST-1L3V was incubated with MBP (lane 1) or MBP fusion proteins containing subfragments of ERp44 (lane 2-7). Binding assay was performed as in (E). The input of MBP and MBP-ERp44 fusion proteins into the binding reaction are shown (top). (F) The effects of expression of ERp44 mutants on IICR in Hela cells in which endogenous ERp44 is knocked down. Peak amplitude of ration is obtained as described in Figure 3D. P<0.005 compared with RFP-RDEL (Student' t test).

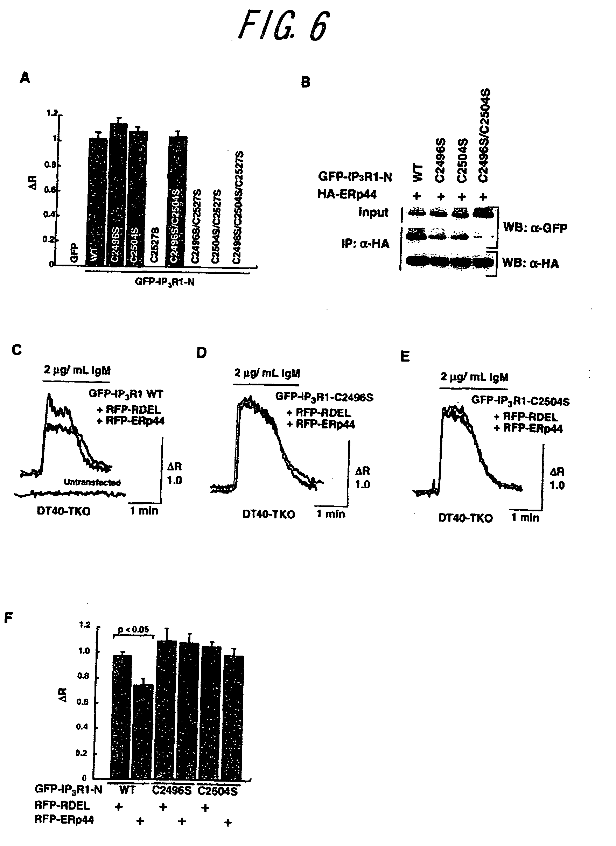

FIG. 6 shows Cysteine Residues of 1L3V are required for the inhibition of IP₃R1 by ERp44. (A) DT40-TKO cells expressing GFP, GFP-IP₃R1, or GFP-IP₃R1 with cysteine replacements were stimulated with 2 µg/mL anti-IgM. Averages of the peak amplitude of Ca²⁺ response are shown from at least three independent experiments (means±SEM), where at least 70 cells were analyzed. (B) Cysteine residues of the L3V domain of IP₃R1 are important for interaction between IP₃R1 and ERp44. GFP-IP₃R1 and its cysteine mutants were transfected with HA-ERp44 in COS-7 cells. Thirty-six hours after transfection, the cells were treated with DSP and then solubilized. HA-ERp44 was immunoprecipitated from the cell lysates. The lysates and immunoprecipitates were analyzed by western blotting with α-GFP (top and middle) or α-HA (bottom). (C-E) ERp44 inhibits IICR in DT 40-TKO cells expressing IP₃R1, but not in DT40-TKO cells expressing IP₃R1 cysteine mutants. Either RFP-RDEL or RFP-ERp44 was transfected into DT40-TKO cells with GFP-IP₃R1-N or its cysteine mutants, respectively. Thirty-six hours after transfection, the cells were stimulated with 2µg/mL anti-IgM. Representative Ca²⁺ responses in RFP-RDEL-transfected (black trace) and RFP-ERp44-transfectd cells (red trace) are shown. (F) Summary of the Ca²⁺ imaging experiments of C-E. Averaged peak amplitude of ration is shown from at least three independent experiments (means±SEM), where at least 50 cells were analyzed.

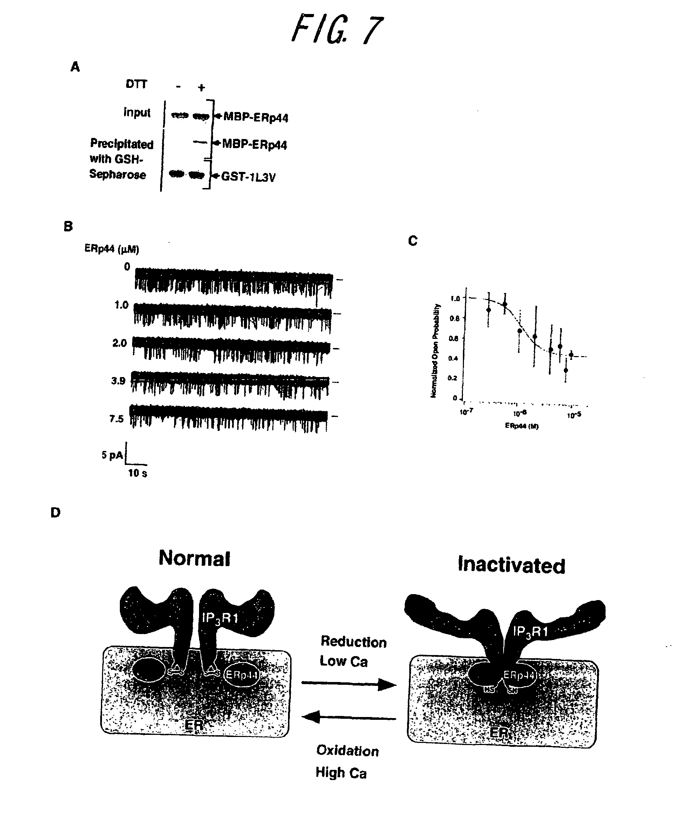

FIG. 7 shows ERp44 inhibits single channel activity of IP₃R1 under the reducing condition. (A) MBP-ERp44 and GST-1L3V were incubated in the trans solution (250mM HEPES (pH 7.33), 53mM Ba (OH)₂)with (+) or without (-) 3mM DTT and then precipitated with GSH-Sepharose. Precipitated proteins were analyzed by western blotting with α-MBP and α-GST. (B) Effect of ERp44 on single channel activity of IP₃R1 in the presence of 6.4 µM cytosolic (cis)IP₃ and 3mM lumenal (trans) DTT. Single channel currents were recorded at 20 mV with 53mM trans Ba2+ as a charge carrier. Purified MBP-ERp44 was sequentially added into the lumenal compartment. The data shown is the representative of six independent experiments. (C) Dose-dependent inactivation of IP₃R1 by ERp44 under the lumenal reducing condition. The averages of IP₃R1 open probabilities were normalized to the maximum IP₃R1 activity observed in each experiment. The data are the means±SD from six independent experiments. (D) Model for the regulation of the channel activity of IP₃R1 by ERp44. When the L3V domain of IP₃R1 is oxidized, ERp44 can not bind to IP₃R1, allowing IP₃R1 to be available for its activation by the agonist stimulation (Normal). On the other hand, the L3V domain of IP₃R1 is reduced, ERp44 is able to interact with IP₃R1, resulting in inactivation of the channel (Inactivated).

### DETAILED DESCRIPTION OF THE INVENTION

A molecular of controlling a calcium according to a first aspect of the invention is (a) an amino acid sequence shown in an amino acid sequence number 1-406 of a sequence No.1 of a sequence table, or (b) an amino acid sequence wherein a part of the amino acid sequence of the amino acid sequence number 1-406 is deleted, substituted or added. Furthermore, a molecular of controlling a calcium according to the invention is (a) an amino acid sequence shown in an amino acid sequence number 1-406 of a sequence No.3 of a sequence table, or (b) an amino acid sequence wherein a part of the amino acid sequence of the amino acid sequence number 1-406 is deleted, substituted or added.

The molecule of controlling a calcium according to the present invention has an ability of specifically binding to IP₃R1 among three types (IP₃R1, IP₃R2, IP₃R3) of an inositol 1,4,5 -triphosphate receptor, allowing IP₃R1 to be inactivate, to reduce the release of a calcium. That is, the present inventions depends on the fact that the inventors found out that a channel activity of IP₃R1 is controlled by pH, redox state, and Lurmenal Ca²⁺ concentration by an effect of ERp44, in a functional interaction between ERp44 and IP₃R1.

Furthermore, a expression vector according to a second aspect of the invention comprises (a) a nucleic acid comprising a base sequence shown in a base sequence number 1-1221 of a sequence No.2 of a sequence table, or (b) a nucleic acid wherein a part of the base sequence of the base sequence number 1-1221 is deleted, substituted or added, and the nucleic acid has a homology of 80% for the base sequence.

Furthermore, a expression vector according to the invention comprises (a) a nucleic acid comprising a base sequence shown in a base sequence number 1-1221 of a sequence No.4 of a sequence table, or (b) a nucleic acid wherein a part of the base sequence of the base sequence number 1-1221 is deleted, substituted or added, and the nucleic acid has a homology of 80% for the base sequence. The above nucleic acid is a nucleic acid encoding a polypeptide which is included in a molecular of controlling calcium according to the presence invention. For example, it is revealed that the use of nucleic acid wherein T of the base sequence number 172 is substituted by A make it possible to control a calcium. And as a result of the above substitution of the nucleic acid, it is also possible to control calcium in case that the use of polypeptide wherein a cysteine of sequence number 58 is substituted by a serine.

If a vector including the nucleic acid is introduced into a cultured cell to be transformed, a polypeptide may be expressed which is include in a molecular of controlling calcium according to the present invention. The cultured cell has an advantage that it is an essential material for experiment in chemical biology, medical science, and pharmaceutical sciences field, and renders substantial contribution.

With respect to the nucleic acids previously described, a purification and isolation method will be described below. The above nucleic acid, for example. can be purified and isolated by the following procedure. A cDNA is synthesized by using a reverse transcriptase after all RNA is extracted by using a kit such as Rneasy Mini Kit (Qiagen) from a cerebella of mouse at the age of 2 weeks. A nucleic acid encoding full length of ERp44 can be obtained by performing a PCR using an artificial composition DNA including a proper base sequence such as a base sequence number 1-21 and 1200-1221 as a primer and a cDNA as a genetic template.

Furthermore, an example of obtaining a polypeptide will be described below. At first, the above nucleic acid is incorporated into a vector including a base sequence encoding a glutachione S-transferase (GST) or maltose binding protein (MBP). After these vector is transformed by using a host such as E. coli, a lot of polypeptide of GST or MBP fused ERp44 (GST-ERp44, MBP-ERp44) can be expressed in E. coli according to stimulation of isopropyl thiogalactoside. After E.. coli is broken by an ultrasonic treatment, GST-ERp44, MBP-ERp44 are specifically bound by the use of Glutathione Sepharose or Amylose Resin, and then it can be specifically eluted by a glutathione solution or maltose solution. At last, a glutathione or a maltose included in the solution is removed by an ultrafiltration, and GST-ERp44 and MBP-ERp44 is purified, and then a polypeptide can be obtained.

The nucleic acid obtained in the way is needed to bind a promoter DNA to express, since it does not have a promoter region which is normally needed for the expression of the nucleic acid. For example, a promoter existed in a vector-plasmid can be used as said promoter. A pcDNA3.1 having a cytomegalovirus (CMV) promoter can be mentioned as a vector having said promoter.

Furthermore, a cultured cell can be transformed by using a recombinant vector obtained in the way. The cultured cell as transformed above has an ability of reducing Ca²⁺ release by means of over-expression of the polypeptide, or an ability of increasing Ca²⁺ release by means of suppressing the expression of the polypeptide.

Furthermore, an antibody of the present invention is against the polypeptide described in claims 1 or 2. An antibody has an ability of being introducing into a cell, and binding a ERp44 to lose its function and controlling Ca²⁺ concentration in a cell.

In a preferable embodiment, the cultured cell according to the present invention can increase an ability of Ca²⁺ release by means of introducing the antibody claimed in claim 5 into a cell, and inducing an antigen-antibody reaction between the antibody and the polypeptide of claims 1 or 3.

A conjunction between ERp44 and IP₃R can be accelerated under existing a reducing agent such as DDT. Furthermore, a binding between ERp44 and IP₃R can be also accelerated under acidic condition (about pH 5.2), or neutral condition such as about pH 7.5 and existing a reducing agent such as DDT.

Moreover, a conjunction between ERp44 and IP₃R depends on a cysteine residue of IP₃R1. That is, a cysteine residue of IP₃R1 play a key role in a suppression of IP₃R1 activity by ERp44, as a result of this, a controlling Ca²⁺ release or suppression can be carried out, depending on whether the cysteine residue exists or not.

Furthermore, ERp44 has an ability of making IP₃R1 inactivate at a low Ca²⁺ concentration so that such Ca²⁺ concentration of ER lumen may be a factor for controlling Ca²⁺ release or suppression.

As mentioned above, a conjunction between ERp44 and IP₃R is controlled by various sorts of factors such as, reducing agent, pH, a cysteine residue, Ca²⁺ concentration. In other words, a conjunction between ERp44 and IP₃R controls a Ca²⁺ release or suppression. According to the mechanism of the above control, a cultured cell having an ability of controlling Ca²⁺ dynamic state can be produced, it can contribute for such as a medical science or pharmaceutical sciences field.

The following examples are given in illustration of the invention and are not intended as limitations thereof. The following examples are intended to illustrate an embodiment of the invention, and it is naturally possible to properly change the invention without departing from the spirit and the scope of the invention claimed in claims.

### Example 1

At first, to reveal the molecular mechanism, we noted a domain having a low homology (L3V) among all subtypes of IP₃Rs existing in ER lumen domain, and examined a protein having an ability of binding these domain. L3V protein of each subtype IP₃Rs fused to an immune globulin Fc was purified and an affinity column was made. A protein with about 40kDa was found which is able to specifically bind to IP₃R1 L3V (1L3V) from a microsome fractionation of a mouse cerebella at age of 2 weeks. It was analyzed by a mass spectrometry, and an amino acid sequence was determined, it was revealed to be homology with ERp44. ERp44 was a protein of ER lumen having a homology to thioredoxin (TRX) which is a redox protein. It is confirmed that ERp44 can bind to IP₃R1 by using immunoprecipitation. To further examine a bind between ERp44 and IP₃R1 in detail, GST pulldown was carried out by using a glutathione S-transferase (GST) fused L3V (GST-L3V) and maltose binding protein (MBP) fused ERp44 (MBP-ERp44) expressed in E. coli and purified. As a result of this, it was found that MBP-ERp44 and 1L3V can directly bind each other in acid buffer including Ca²⁺. Next, it was also revealed that ERp44 and 1L3V can bind under a neutral condition including a reducing reagent dithiothreitol (DTT). Furthermore, the bind depended on a cysteine residue of 1L3V and Ca²⁺ concentration. Furthermore, it was confirmed that only ERp44 can bind to IP₃R1 among three subtypes of IP₃Rs. These experiment suggests that ERp44 can specifically bind to IP₃R1 by detecting a redox state of ER and Ca²⁺ concentration.

Next, it was examined how effects a ERp44- IP₃R1 bind gives a channel activity in vivo according to a Ca²⁺ imaging experiment using various cells. A red fluorescent protein (RFP) fused ERp44 (RFP-ERp44) was expressed in a cell, and an agonist ATP stimulation of a IP₃ production was carried out. IP₃ -induced Ca²⁺ release (IICR) significantly reduced in HeLa cell which expresses RFP-ERp44 (IP₃R1 among subtypes was predominantly expressed.).

Further, in a COS-7 cell (IP₃R1 was hardly expressed.), an expression of RFP-ERp44 has no affect to IICR. A result of Ca²⁺ imaging experiment using these HeLa and COS-7 cells and a bind experiment in vitro strongly suggest that ERp44 can bind only IP₃R1 among all subtypes and suppress their activity. To further confirm this, B lymphocyte cell type DT40 of a chicken was used. Taking advantage of a characterization of this cell, cells which all subtypes of IP₃R are lost (DT40-TKO cell) and, a cell which only IP₃R1 is lost (DT40-1KO cell) were made. Furthermore, a cell wherein IP₃R1 is expressed in DT40-TKO cell (DT40-KMN60) is made. IICR was examined after BCR was cross-linked with IgM antibody. A IICR significantly reduced in cells which express RFP-ERp44, but it was not observed in DT40-1KO cell about an affect of IICR. This result suggests that ERp44 can suppress IICR through not IP₃R2 or IP₃R3, but IP₃R1.

To further examine a function of endogenous ERp44, an experiment was carried out by using RNA interference (RNAi). In a HeLa cell wherein knockdown of ERp44 is carried out, a ratio of a cell in which IICR is induced by ATP stimulation and an intensity of Ca²⁺ release are significantly increased. On the other hand, in COS-7 cells, knockdown of ERp44 had no effect on IICR. These loss-of-function experiment strongly suggests that ERp44 can suppress IP₃-induced Ca²⁺ release via IP₃R1, but not IP₃R2 or IP₃R3. Further, this result also suggests a result obtained by gain-of-function experiment.

To further confirm the result obtained from RNAi, ERp44 was re-expressed in a knockdown cell of ERp44. An expression of ERp44 significantly reduced a IICR. As a result of this, it was concluded that ERp44 can inhibit IP₃R1. Furthermore, we examined how domain of ERp44 is important for inhibition of IP₃R1 by using this re-expressed lines. At first, it was found that an amino acid from 236 to 345 of ERp44 is IP₃R1 binding domain (IPBD). Surprisingly, used mutants, however, even if it was a IPBD, were not able to inhibit IP₃R1 compared with that of full lengths of ERp44. These results suggest that an inhibition of IP₃R1 requests not only a binding domain but other domain except for said binding domain. Next, an importance of a cysteine residue is examined. A binding between cysteine mutant of IP₃R1 which is expressed in COS-7 cells, and ERp44 significantly reduced. To examine a physiological means, a cysteine mutant of IP₃R1 and RFP-ERp44 were expressed in DT40-TKO, and they were stimulated by IgM antibody, but an inhibition of IICR was not observed. According to this result, it was found that a cysteine residue is necessary to inhibit IP₃R1 activity by ERp44.

Furthermore, to examine how affect on channel activity according to a bind between ERp44 and IP₃R1, a measurement of IP₃R1 activity on single-channel level was carried out by using an artificial lipid bilayer system. This in vitro reconfiguration system allows us to assess how affect on IP₃R1 activity induced by ERp44 since Ca²⁺ concentration of ER lumen and redox state can be strictly controlled. As a result, ERp44 made IP₃R1 inactivation under conditions of an existence of DTT and a low Ca²⁺ concentration. This result clearly suggests that ERp44 can detect a redox state and Ca²⁺ concentration of ER to inhibit IP₃R1 specifically.

According to the fact mentioned above, a new mechanism of controlling Ca²⁺ signaling was found by using a manner performed in a chemical biology, a biochemistry, a cell biological study, and an electrophysiology.

A detailed explanation will be below.
An inositol 1, 4, 5 triphosphoric acid receptor (IP₃Rs) is a internal channel protein and relates to a number of biologic processes and disorders wherein a medium Ca²⁺ is extracted from endoplasmic reticula (ER). IP₃Rs is regulated by distinguishing various cytosol proteins, it is still unsolved about a regulation of ER lumen proteins. We found that ERp44, that is, ER lumen proteins of thioredoxin family, directly interacts with the third lumen loop of IP₃R (IP₃R1), and this interaction depends on pH, redox state, and Ca²⁺ concentration of ER lumen. A Ca²⁺ imaging experiment of IP₃R1 and single channel record using lipid bilayer system clearly demonstrate an direct inhibition of IP₃R1 by ERp44. These results strongly suggested that ERp44 is a micro environment of ER lumen and it was translated into a cytosol Ca²⁺ concentration by means of controlling IP₃R1.

Introduction
In this example, to elucidate the molecular mechanism of type-specific regulation of IP₃Rs from the ER lumen, we searched for binding proteins of the L3V domain of IP₃Rs. We then identified ERp44 a protein of thioredoxin family preciously implicated in oxidative protein folding, as a specific binding protein with the L3V domain of IP₃R1. The unique characteristics of the functional interaction between ERp44 and IP₃R1 suggest that the channel activity of IP₃R1 is modulated by pH, redox state, and [Ca²⁺]ER by virtue of ERp44.

Results
Identification of ERp44 as a Binding Protein of the L3V Domain of IP₃R1
We aimed to find proteins that bind to the L3V domain of IP₃Rs. Yeast two-hybrid screening, which has been successful for identification of IP₃Rs-binding proteins in cytosol, was considered not to be appropriate for this purpose, since chemical environment of nucleus is completely different from that of ER lumen. We therefore, made the 1L3V-Fe, in which secretion signal and human immunoglobulin Fe domain were fused to the N-and C-terminus, respectively, of the L3V domain of IP₃R1 (1L3V). This protein was expressed in 293T cells, collected from the medium, and utilized to make affinity resin. Initially, we failed to detect any 1L3V-Fc-interacting protein from mouse cerebellar microsomal fraction solubilized in neutral buffer (pH 7.5, data not shown). Then, we performed the similar experiment under acidic conditions (pH 5.2), as chromogranin A binds to the L3C domain of IP₃R only under this condition. Consequently, we identified two proteins that bound to 1L3V-Fc, but not to control-Fc (Figure 1C). We excised these bands and analyzed them by mass spectrometry. Two peptide sequences, HPLLHIQK and QFVFDLHSGK, were obtained from the 44kDa protein (Figure 1C), indicating that this protein is ERp44 (Anelli et al). The 85kDa protein was identified as acoonitase, a mitochondrial protein, and was not further characterized in this study. Using the same procedures, we also searched for bindings protein for the L3 domain of IP₃R2 or IP₃R3 (2L3V or 3L3V, respectively) from mouse brain, but we have been unable to identify any so far.

ERp44, an ER lumenal protein of chioredoxin (TRX) family, contains a signal peptide (SP), TRX-homology domain (THD), and an ER retention signal, RDEL (Figure 1D) (Anelli et al, 2002). ERp44 is widely expressed in mouse tissues (Figure 1). Haemagglutinin A epitope (HA)-tagged ERp44 (Anelli et al, 2002) was expressed in COS-7 cells, either alone or with IP₃R1 fused N-terminally with green fluorescent protein. HA-ERp44 was observed as a diffusive network, as GFP-IP₃R1 and endogenous CRT, indicating that ERp44 colocalizes with IP₃R1 in the ER. To verify interaction between IP₃R1 and ERp44, HA-ERp44 and IP₃R1 were expressed in COS-7 cells. We first found that both HA-ERp44 and IP₃R1 were severely degraded when whole cell lysate was prepared with acidic buffer (in which association was observed in experiments shown in Figure 1C), presumably by lysosomal proteases. To circumvent this, the transfected cells were treated with a membrane-permeable crosslinker dithiobissuccinimidylpropionate (DSP), and solubilization and immunopresipitation was performed in neutral buffer. Under this condition, anti-IP₃R1 antibody coprecipitated HA-ERp44 and, reciprocally, anti-HA antibody coprecipitated IP₃R1 (Figure 1F), indicating that ERp44 and IP₃R1 are in a same complex in transfected COS-7 cells. To examine endogenous association, immunoprecipitations were performed from whole cell lysate of untransfected HeLa cells, which expressed IP₃R1 at relatibely high level. Coprecipitation between ERp44 and IP₃R1 was clearly observed, indicating that they are in a same complex in vivo (Figure 1G).

ERp44 specifically interact with IP₃R1, but not with IP₃R2 or IP₃R3, in condition-dependent manner.
To further characterize association between ERp44 and IP₃Rs, we next performed series of pull-down experiments. Glutachione (GSH) S-trandferase (GST) and maltose binding protein (MBP) were fused to 1L3V and ERp44, respectively (Figure 2A), and these proteins were expressed in E. coli and purified. Under acidic condition (pH 5.2), MBP-ERp44, but not MBP alone, interacted with GST-1L3V, but not with GST (Figure 2B). This interaction, however was not observed under neutral (pH 7.2) condition (data not shown). The pH of the ER lumen was estimated to be near neutral in most of the literatures (e.g. Kim et al., 1998; Foyouzi-Youssefi et al., 2000). We reasoned that certain factor might have been missed in our pull-down assays, and next sought for conditions under which 1L3V and ERp44 interacts at neutral pH. The presence of THD in ERp44 suggests that the function of this protein is redox-dependent, and it was reported that ERp44 forms mized disulfide bonds with a number of proteins (Anelli et al., 2003; Anelli et al., 2002). Thus, we examined whether interaction between 1L3V and ERp44 was dependent on the redox state. Both GST-1L3V and ERp44 were mostly in their oxidized forms in the absence of a reducing reagent, dithiothreitol (DTT), as judged by their migration pattern on SDS-PAGE under non-reducing condition. Interaction between these oxidized forms was not observed at pH7.5 (Figure 2C, lane 1, middle panel). Addition of DTT reduces GST-1L3V and ERp44 and, strikingly, it was found that reduced forms of these proteins were able to interact with each other at pH7.5 (Figure 2C, lane 2). It was also revealed that this interaction is not caused by mixed disulfide bonds since no high-molecular weight band (i.e. covalently linked GST-1L3V-ERp44) was observed (data not shown). To investigate redox state of which protein is important for this interaction, the cysteine residues of these proteins were mutated. Regarding 1L3V, mutation of either Cys2496, Cys2504, or Cys2527, led to decreased binding to recombinant ERp44 (Figure 2D). By contrast, none of ERp44 cysteine mutations affected their binding to GST-1L3V, but none of ERp44, are important for the interaction between ERp44 and 1L3V was required for interaction between 1L3V and ERp44.

We also examined whether the interaction between ERp44 and 1L3V is dependent on Ca²⁺ concentration. As shown in Figure 2E, the interaction diminished when Ca²⁺ concentration was higher than 100µM. The [Ca²⁺]ER was estimated to be roughly between 200 µM and 1mM under resting conditions (Meldolesi and Pozzan, 1998), suggesting that interaction between ERp44 and 1L3V is not very robust at resting state and is enhanced when [Ca²⁺]ER decreased (e.g. after Ca²⁺ release).

The above findings suggested that interaction between ERp44 and IP₃R1 favors reducing and/or low [Ca²⁺]ER conditions. To examine this in vivo, COS-7 cells expressing GFP- IP₃R1 and HA-ERp44 were treated with 5mM DTT, and then HA-ERp44 was immunoprecipitated (Figure 2F). Although HA-ERp44 precipitated with anti-HA antibody was consistently diminished under reducing conditions for unknown reasons (Figure 2F, bottom panel), GFP-IP₃R1 co-precipitated with HA-ERp44 was significantly increased (Figure 2F, middle panel). Next, CoS-7 cells epressing HA-ERp44 an acivator of purinergic receptors. This treatment increased GFP-IP₃R1 that was co-precipitated with HA-ERp44 by approximately 2-folds, indicating that lowering [Ca²⁺]ER augments association between IP₃R1 and ERp44. These results indicated that the dynamic change of the ER luminal environment controls interaction between IP₃R1 and ERp44. Finally, we found that MBP-ERp44 interact with GST-1L3V, but neither with GST-2L3V nor GST-3L3V, which is consistent with the results of initial screening using L3V-Fc (Figure 1). Take together, it was concluded that ERp44 directly interacts with the L3V domain of IP₃R1, in a subtype-specific and the ER lumenal environment manner.

Expression of ERp44 inhibits IICR via IP₃R1.
To explore the functional consequence of binding of ERp44 to IP₃R1, we carried out single-cell Ca²⁺ imaging experiments using a fluorescent Ca²⁺ indicator fura-2. First, we employed HeLa cells, in which IP₃R1 constitutes about half of total IP₃Rs, and association between IP₃R1 and ERp44 is observed (Figure 1G). Red fluorescent protein (RFP)-tagged ERp44 (RFP-ERp44) or RFP with an ER retention signal (REP-RDEL, negative control) was expressed in HeLa cells, and they were stimulated with ATP in nominally Ca²⁺-free medium. In the following experiments, Ca²⁺ signals between transfected (RFP signal-positive) and non-transfected (RFP signal-negative) cells in a same dish were compared. In non-transfected cells, ATP (3 µM) typically caused a single Ca²⁺ transient occasionally with small oscillations (Figure 3A black trace). HeLa cells expressing RFP-RDEL showed indistinguishable responses Figure 3A, red trace). On the other hand, HeLa cells expressing RFP-ERp44 typically shown significantly smaller Ca²⁺ transient (Figure 3B, red trace). On average, the peak amplitude in cells expressing RFP-ERp44 was significantly decreased (69.3±1.8% of that of non-transfected cells; p<0.05, Figure 3C). This was not due to the reduction of releasable Ca²⁺ content in the ER, as a passive Ca²⁺ leak elicited by thapsigargin (Tg, a SERCA inhibitor) was unchanged (Figure 3D, 3E). These results indicated that ERp44 inhibited IICR without affecting Ca²⁺storage capacity in HeLa cells.

We next tested the effect of ERp44 overexpression in COS-7 cells that virtually expressed no IP₃R1 (Boehning and Joseph, 2000; Hattori et al., 2004). In non-transfected cells and cells expressing RFP-RDEL, stimulation with 1 µM ATP elicited a Ca²⁺transient. Strikingly, overexpression of RFP-ERp44 did not affect the amplitude of the transient (Figure G). The averaged amplitudes in cells expressing RFP-RDEL and RFP-ERp44 were 98.6±0.7 % and 104±2.5 % of those in non-transfected cells, respectively (n=60 and 80; Figure 3H). The effect of RFP-ERp44 overexpression was not observed when cells were stimulated with weaker stimulation (0.3 µM ATP), too (data not shown). These results indicated that ERp44 did not affect IICR in COS-7 cells. The above results suggested that ERp44 inhibits IP₃R1, but not IP₃R2 or IP₃R3. To further confirm this, we employed two more cell lines, DT40-KMN60 was established by stably-transfecting mouse IP₃R1 gene on the background of DT40 cells in which all the IP₃R genes were disrupted (DT-TKO, Sugawara et al., 1997). DT40-1KO cells express in production of IP₃R2 and IP₃R3 , but not IP₃R1, since the gene for IP₃R1 was disrupted (Sugawara et al., 1997). Crosslinking of B-cells antigen receptor (BCR) activates PLC-γ, resulting in production of IP₃ in DT40 cells. In DT40-KMN60 cells, expression of RFP-RDEL did not affect BCR-induced Ca²⁺release (96±2.9%) compared with non-transfected cells. On the other hand, expression of RFP-ERp44 decreased the peak amplitude of BCR-induced Ca²⁺release in DT40-KMN60 cells (60.6±9.6%, Figure 3I and 3K)). There was no reduction in Tg-induced Ca²⁺leak in these cells (Figure 3L and 3M). In DT40-1KO cells, on the other hand, expression of RFP-ERp44 had little effect on BCR-induced Ca²⁺ release (Figure 3J and 3K). These results provide strong evidence that ERp44 inhibits IP₃R1, but neither IP₃R2 or IP₃R3.

Since function of IP₃Rs is known to be critical for BCR-induced apoptosis in DT40 cells (Sugawara et al., 1997), we next examined whether expression of ERp44 affects it. Expressing RFP-ERp44, but not RFP-RDEL, significantly inhibited apoptosis in DT40-KMN60 cells (Figure 3N and 30). On the other hand, this effect was not observed in DT40-1KO cells (Figure 30). These results implied that modulation by ERp44 on IP₃R1 affects cellular function such as apoptosis.

Specific knockdown of ERp44 results in augmentation of IICR via IP₃R1 To investigate roles of endogenous ERp44, we next employed the RNA interference technique. We tested two different small interfering RNA (sRNA) sequences that were rargeted to either the open reading frame (siERp44-ORF) or the 3'-untranslated region (siERp44-3U), respectively, of human ERp44. As negative controls, we introduced three-point mutations in these siRNAs (siControl-ORF or siControl-3U, respectively). Western analysis revealed that both siERp44-ORF and siERp44-3U efficiently (>90%) decreased expression of ERp44 without affecting that of actin, IR3R1, and IR₃R3 in HeLa and COS-7 cells. Unfortunately, no siRNA targeted to chicken ERp44 decreased expression of ERp44 in DT40 cells (data not shown).

SiRNA-rtansfected HeLa cells were stimulated with 1, 3, 10 µM ATP. In siControl-3U-transfected cells, 1 µM ATP rarely (4.0±0.6% of total cells, n=201) evoked discernible Ca²⁺ signals (Figure 4B and 4C, black trace and bar). On the other hand, the number of responding cells was higher (20.1±4.9%, n=213) in siERp44-3U-transfected cells by approximately 5-folds (Figure 4B and 4C, red trace and bar). Subsequent stimulation of 3 µM ATP elicited IICR in 46.9±3.1%, of control cells and 90.3±2.4% of cells transfected with siERp44-3U (Figure 4B and 4C). When stimulated with 10 µM ATP, most of cells responded in either preparation (96.9±1.9% and 99.6±0.4%, respectively; Figure 4C). The averaged peak amplitude was higher in siERp44-3U-transfected cells than in siControl-3U-transfected cells under all ATP concentrations (Figure 4D). Knockdown of ERp44 did not affect Ca²⁺ leak induced by Tg (data not shown). These results indicated that the endogenous ERp44 negatively regulates IICR in HeLa cells. These results indicated that the endogenous ERp44 negatively regulates IICR in HeLa cells.

Next, we performed the same experiments in COS-7 cells. As COS-7 cells do not express IP₃R1, our prediction was that knockdown of ERp44 had no effect on IICR in this cell line, and it was indeed the case (Figure 4E-4G). These results support the idea that ERp44 inhibits only IP₃R1.

As a further confirmation that the effect of ERp44 knockdown was specific, we " restored "expression of ERp44 in siRNA-transfected cells. HeLa cells were transfected with either sicontrol - 3U or siERp44-3U and 24 hours later the cells were transfected with the expression vector for RFP-RDEL or RFP-ERp44. As siERp44-3U targets the 3'-UTR sequence that is absent in the expression vector for RFP-ERp44, expression of endogenous ERp44, but not that of RFP-ERp44, was suppressed by siERp44-3U (Figure 5A, lane 4). Following these treatments, the cells were stimulated with 3µM ATP. In siERp44-3U-pretreated cells (i.e. the amplitude of IICR was increased),restoration of RFP-RDEL had little effect on IICR (Figure 5B), while that of RFP-ERp44 significantly suppressed it (Figure 5C). Altogether, it was concluded that ERp44 specifically inhibits IP₃R1.

Furthermore, this system allowed us to assess which domain of ERp44 is important for inhibition of IICR First, minimum 1L3V-interacting region of ERp44 was determined in vitro (Figure 5D and 5E). Full-length (without the N-terminal signal sequence) or subfragments of ERp44 were fused to MBP and their interaction with GST-1L3V was tested in the presence of DTT at neutral pH. Wild-type MBP-ERp44 and MBP-ERp44-a (C-terminal half) strongly interacted with GST-1L3V (Figure 5E, lanes 2 and 3,respectively), indicating that THD is dispensable for the interaction. MBP-ERp44-b,which contains highly conserved glutamate-rich region but excludes histidine-rich region, also bound to GST-1L3V, albeit somewhat weakly (Figure 5E, lane 4), whereas other mutants tested did not (Figure 5E, lanes 5-7, respectively). These results indicated that amino acid residue 236-345 of ERp44 was necessary and sufficient for binding to 1L3V, and hereafter referred as IP₃R1 binding domain (IPBD). The primary sequence of IPBD has no similarity to any sequence in the database, but is well conserved among species (Figure 3).

Then, HeLa cells, pretreated with siERp44-3U, were transfected with various RFP-tagged ERp44 mutants and their ability to inhibit IICR was examined (Fig. 5F). RFP-ERp44-f, which retains THD but not IPBD, had no effect. Interestingly, neither RFP-ERp44-a nor RFP-ERp44-b-RDEL, both of which retain IPBD, inhibited IICR at all (Figure 5F, lanes 5 and 6), indicating that IPBD alone is not sufficient for inhibition of IP₃R1. Moreover, RFP-ERp44-C58S, in which the cysteine residue critical for formation of mixed disulfide bonds with other proteins is mutated (Anelli et al., 2003),only slightly inhibited Ca²⁺ response (77.1±4.5%, n=82, not statistically significant)compared with RFP-RDEL-transfected cells (Figure 5F, lane 3). These results indicated that both THD (including Cys58) and IPBD are required to inhibit IP₃R1 activity.

Cysteine residues in 1L3V play crucial roles for inhibition of IP₃R1 channel activity by ERp44 To examine the significance of cysteine residue(s) of 1L3V, we constructed expression vectors for GFP-IP₃R1 in which the cysteine residue in L3V region was substituted for serine, either singly or in combinations. Each vector was transfected to IP₃R-deficient DT40-TKO cells and its IICR activity was checked. As a result, it was revealed that substitution of C2527, which is adjacent to the channel pore region, impairs the channel activity of IP₃R1 (Figure 6A), and thus this mutant was not used further. Mutations in C2496 and C2504 were without effect on the channel activity (Figure 6A). Interactions between these cysteine mutants and ERp44 were significantly decreased (Figure 6B),consistent with the results using recombinant proteins (Figure 2D). These mutants were co-transfected to DT40-TKO cells either with RFP-RDEL or with RFP-ERp44, and single-cell Ca²⁺-imaging experiment was performed. IICR via GFP-IP₃R1 (i.e. wild-type) was suppressed by co-expression of RFP-ERp44, but not with RFP-RDEL (Figure 6C and 6F). Surprisingly, this inhibition by ERp44 was almost completely abolished by substitution of C2496 or C2504 of GFP-IP₃R1 (Figure 6D and 6E, respectively, and Figure 6F). This could be explained by reduced affinity between these mutants and ERp44, or alternatively, it is possible that both cysteine residues are required for inhibition by ERp44, which is supported by the fact that both IPBD and Cys58 of ERp44 were required for inhibition of IP₃R1 (Figure 5F). In any case, these results clearly demonstrated that the presence of free thiol groups in 1L3V is important for inhibition by ERp44.

ERp44 inactivates the channel activity of IP₃R1 in the lipid bilayer system. To unambiguously show that ERp44 inhibits IP₃R1, we carried out single-channel currents recoding in planar lipid bilayer fused with mouse cerebellar microsome, in which more than 99% of IP₃R is IP₃R1. We first confirmed interaction of MBP-ERp44 with GST-1L3V in the lumenal-side solution containing 3 mMDTT (Figure 7A). IP₃R1 activity was recorded with Ba²⁺. as a charge carrier in the presence of 3 mM DTT in the trans (lumenal) compartment. Under this condition, IP₃R1 was maximally activated by addition of 6.4 µMIP₃ to the cis (cytosolic) compartment (data not shown). Addition of MBP (negative control) to the lumenal compartment had no effect on IP₃R1 activity (data not shown). In contrast, addition of MBP-ERp44 resulted in inhibition of the channel activity in a dose-dependent manner (Figure 7B). Single-channel open probability was reduced with IC50 of 1.0 µM MBP-ERp44 and with a Hill coefficient of 2.3 (Figure 7C), suggesting that binding of at least two ERp44 molecules to a heterotetramer of IP₃R1 is required for inactivation. Very interestingly, this inactivation of was not observed in the absence of DTT (data not shown), further supporting the idea that the free thiol group(s) in 1L3V is involved by inhibition by ERp44. The mean open time, current amplitude, and conductance of IP₃R1 were unaffected by addition of MBP-ERp44 (data not shown), revealing that the function of ERp44 is to keep the closed state of IP₃R1 , rather than affecting the characters of its open state.

Discussion
In this study we found that ERp44, an ER lumenal protein, directly interacts with the L3V domain of IP₃R1 and thereby inhibits its channel activity. This functional interaction is dependent on pH, the redox state, and [Ca²⁺]ER. This is the first example of a negative regulator of IP₃Rs in the ER lumen, embodying the idea that IP₃Rs are specifically and temporally controlled by the intralumenal environment.

Among pH, the redox state, [Ca²⁺]ER, which of the factors is most dominant (or important) in regulation of ERp44/IP₃R1 interaction? This question relates to how intralumenal environment of the ER is regulated and/or subject to change by cytosolic or extracellular conditions. A number of works have been conducted to elucidate the intralumenal environment of the ER, but the conclusions drawn from them are not necessarily consistent. Regarding pH, however, there seems to be a consensus that it is around neutral and stable, even during or after Ca²⁺ release (Foyouzi-Youssefi et al., 2000; Kim et al., 1998). From these observations, we do not favor the idea that pH plays major roles in regulation of ERp44/IP₃R1 interaction in vivo. It is, however, still possible that pH changes drastically (perhaps only for a very short period of time) in the neighbor of the ER membrane, or the change of pH plays a role in the sarcoendoplasrmic reticulum or secretory granules within which pH could be acidic (Kamp et al., 1998;Quesada et al., 2003).

It is widely accepted that the ER lumen is more oxidizing than cytosol.
In the ER lumen, the ratio of reduced GSH to oxidized one has been estimated to be 3:1 to 1:1 (Bass et al.,2003; Hwang et al., 1992). Cysteine residues of proteins that are destined for the extracellular milieu are mostly oxidized to form disulfide bonds by virtue of ER oxidoreductases. Molecular mechanisms by which the redox status of extracellular proteins and ER oxidoreductases are controlled are becoming clear (Sevier and Kaiser, 2002), but it remains unexplored how the redox status of other ER resident proteins (including IP₃Rs) are regulated (if any). Our results indicated that interaction between ERp44 and 1L3V becomes weaker as the number of free thiol group on 1L3V decreases (Figures 2D, 2F, and 6B). Furthermore, the inhibitory effect of ERp44 on IP₃R1 is rather partial in systems where contribution of other IP₃R subtypes are excluded (Figures 3I,6C, and 7C), implying that not all of IP₃R1 is subject to inhibition by ERp44. Altogether, we propose that some of the cysteine residues in 1L3V are in their free (reduced) form (i.e. subject to ERp44 inhibition), and others form disulfide bonds, either intramolecularly or intermolecularly (i.e. no ERp44 interaction or inhibition). It is likely that the balance between these statuses is tightly regulated (by virtue of the ER oxidoreductases, for example). It is well known that sulphydryl-oxidizing reagents (such as thimerosal) reversibly sensitizes IICR in many cell types including HeLa cells (Bootman et al., 1992; Karhapaa et al., 1996; Montero et al., 2001). Furthermore, IP₃R1 ,but not IP₃R3, is sensitized by thimerosal treatment (Missiaen et al.,1998). These phenomena may be the reflection of the redox-dependent inhibitory effect of ERp44 on IP₃R1.

Previous studies showed that IICR was diminished at low [Ca²⁺]ER, which could not be fully explained by the decreased gradient of Ca²⁺ concentration across the ER membrane (e.g. (Caroppo et al., 2003)). Our biochemical result (Figure 2E) indicated that the interaction between ERp44 and IP₃R1 is weakened when [Ca²⁺]ER is more than 100 µM (i.e. at resting level). It is therefore tempting to speculate that the observed inhibition of IICR at under low [Ca²⁺]ER conditions is due to enhanced binding by ERp44 to IP₃R1. Such a mechanism may be effective for preventing [Ca²⁺]ER from becoming too low for other intralumenal proteins (such as Ca²⁺-dependent enzymes and chaperones) to exert their functions.

What is the physiological significance of inhibition of IP₃R1 by ERp44? One possibility is to translate the ER lumenal environment into [Ca²⁺]c. The ER is the crosspoint of many signaling pathways (Berridge, 2002; Orrenius et al., 2003). Therefore, inhibition of IP₃R1 by ERp44 may be a part of the feedback system by which information converged at the ER was conveyed to the cytosol/nucleus. Another possibility is to prevent [Ca²⁺]ER from becoming too low for other ER lumenal proteins (such as Ca²⁺-dependent enzymes and chaperones) to exert their functions. ERp44 was first identified as a protein that forms mixed disulfide bonds with Erolα, an ER oxidoreductase (Anelli et al., 2002), and it was shown to be involved in its ER retention (Anelli et al., 2003). The detailed function of Erol family proteins are not full understood, but it plays a pivotal role in oxidative protein folding in the ER lumen (Tu and Weissman, 2002).

ERp44 overexpression shifts the redox status of Erolα to oxidized form and thus is thought to promote oxidative folding (Anelli et al., 2002). Therefore, ERp44 may play dual roles in protein folding: inhibiting Ca²⁺ release (which reinforces Ca²⁺-dependent chaperones) by inactivating IP3R1 and supporting disulfide bond formation by boosting Ero1α/oxidoreductase system. This idea is further supported by the fact that ERp44 is induced during the unfolded protein responses (Anelli et al., 2002).

It was also previously shown that ERp44 forms mixed disulfide bonds with many proteins, including integral membrane proteins (Anelli et al., 2003; Anelli et al., 2002). At present it is unknown whether ERp44 forms mixed disulfide bonds with IP₃R1. The pull-down assays using recombinant proteins revealed that IPBD is sufficient for interaction between these proteins. On the other hand, interaction between ERp44-C58S and IP₃R1 in transfected cells was diminished (Supp), and ERp44-C58S is less competent in inhibiting IP₃R1 (Figure 6F). These results suggest that C58 plays some role in its interaction with IP₃R1 in vivo. However, another interpretation of these ,results is that because ERp44 (but not ERp44-C58S, (Anelli et al., 2003)) can form a homodimer with disulfide bonds in vivo, interaction of one ERp44 molecule may bring another molecule that co-precipitates and inhibits IP₃R1. In any case, further studies will be required to understand the exact mechanism of ERp44's action. It is unlikely that IP₃R1 is retained in the ER by virtue of ERp44 since the L3V domain of IP₃R1 is dispensable for the ER retention (Galvan et al., 1999) and IP₃R1 is still localized to the ER in cells in which ERp44 was knocked down (data not shown).

It is also mentioning that while most of the biochemical characteristics and functions of Erol are conserved between yeast and mammals, yeast does not have an ERp44 orthologue (Anelli et al., 2003). Yeast does not have an IP₃R, either, and C. elegans has both ERp44 and IP₃R, suggesting that these proteins may have emerged at similar time points in the course of evolution.

Very recently, it was reported that the activity of SERCA 2b is modulated by CRT and ERp57, an ER lumenal oxidoreductase, in [Ca²⁺]ER- and redox - state dependent manner (Li and Camacho, 2004). The pump activity of SERCA 2b is higher when thiol groups in its lumenal loop are reduced. Thus, when the ER lumenal environment shifts to the reducing condition, Ca²⁺ uptake by SERCA 2b would be enhanced (by ERp57) and Ca²⁺ - release via IP₃R1 would be inhibited (by ERp44). Moreover, reduced ER lumenal environment is not favorable for protein folding. Thus, it is reasonable to increase [Ca ²⁺]ER by inhibiting IP₃R1 and activating SERCA 2b since function of many chaperones require relatively high [Ca²⁺]ER.

Finally, although only IP₃R1, not IP₃R2 or IP₃R3, is regulated by ERp44, it does not necessarily mean that IP₃R2 and IP₃R3 are insensitive to the ER lumenal environment. We were unable to find any binding protein to the lumenal domain to IP₃R2 or IP₃R3 from the brain (where IP₃R1 is exclusively expressed), but it is likely that these subtypes have different binding proteins that are absent in the brain. The fact that cysteine residues in the L3V domain are conserved (Figure1B) supports this hypothesis.

### Experimental procedures

### Plasmids

Full-length ERp44 cDNA was obtained by reverse transcriptase-polymerase chain reaction from P14 mouse cerebellum. The expression vectors for epitope-tagged or fluorescent protein-tagged ERp44, IP₃Rs and their mutants were constructed by utilizing polymerase chain reaction. Detailed methods, including the sequence of primers used, will be supplied on request. All constructions were verified by sequencing.

### Antibodies

Rabbit polyclonal antibody against ERp44 was raised against purified His6-ERp44 and affinity-purified with MBP-ERp44 coupled to CNBr-activated Sepharose 4B (Amersham). Other antibodies used were anti-IP₃R1 (18A10, (Maeda et al., 1988)),Anti-IP₃R3 (KM1082, (Sugiyama et al., 1994)), anti-HA (12CA5) gifted from Dr.Tadashi Yamamoto, anti-HA-Peroxidase (3F10, Roche), anti-CRT (Affhity BioReagents), anti-MBP (New England Biolabs), anti-human IgG (Vector Laboratories),anti-GST (Amersham), anti-GFP, and anti-actin (Santa Cruz). The hybridoma producing anti-IgM (M-4 clone) was kindly provided by Dr. Tomohiro Kurosaki (Kansai MedicalUniversity).

### Cell Culture and Transfection

COS-7, HeLa, and 293T cells were cultured in Dulbecco's modified Eagle medium supplemented 10% heat-inactivated fetal bovine serum. DT40 cells were cultured in RPMI1640 medium supplemented 10% heat-inactivated fetal bovine serum, 1% chicken serum, 100 U/mL penicillin and streptomycin, 2 mM glutamine and 50 µM 2-mercaptoethanol. HeLa and COS-7 cells were transfected with expression vectores or siRNAs using TransIT (Mirus) or Lipofectamine2000 (Invitrogen), respectively, as described previously (Hattori et al., 2004). DT40 cells were transfected with expression vectors using Nucleofector (Amaxa).

### Identification of ERp44

1L3V-Fc or control Fc was expressed in 293T cells. The medium was collected and incubated with Protein-G Sepharose (Amersham) for 2 hr at 4°C and then the beads were washed with 10 mM Tris-HCl (pH 8.0), 150 mM NaCl. Approximately 0.2 mg of 1L3V-Fc or control Fc was coupled to the beads, which was laid onto a column. P14 mice cerebella were washed with ice - cold PBS and homogenized in a buffer containing 0.32 M sucrose, 10 mM Tris-HCl (pH 7.5), 2 mM EDTA, and 1 mM DTT. The homogenates were centrifuged for 1hr at 100,000 x g at 2°C. The pellet was solubilized in buffer containing 30 mM sodium acetate, 150 mM NaCl, 4 mM CaCl2, 1% TritonX-100 for 1 hr at 4°C and centrifuged at 10,000 x g for 15 min. The supernatant was applied to the affinity columns. The columns were washed with the same buffer and then neutral Ca²⁺ buffer (10 mM Tris-HCl (pH 7.5), 150 mM NaCl, 4 mM CaCl2, and 1 % TritonX-100), and then neutral cherators buffer (10 mM Tris-HCl (pH 7.5), 150 mM NaCl, 4 mM EDTA, 4 mM EGTA, and 1%TritonX-100). Fractions were collected and resolved in SDS-PAGE. After silver staining of the gels, the bands were excised and analyzed as described previously (Ichimura et al., 2002).

### Western blot analysis

Proteins were resolved by SDS-PAGE and transferred to a polyvinylidene difluoride membrane. Membranes were blocked with 5% skim milk in PBS including 0.2% Tween-20 (PBST) for 30 min and probed with the first antibody for 3 hr at room temperature (RT). After washing with PBST, membranes were incubated with a suitable secondary antibody for 1hr, and signals were detected using ECL Plus kit (Amersham).

### Immunostaining

Cells were fixed with 49% formaldehyde for 10 min at RT and permeabilized with 0.2 %Triton X-100 in PBS for 10 min at RT. After blocking in PBS including 5% goat serum for 1hr at RT, the cells were incubated with first antibodies for 3 hr at RT. Followed by incubation with suitable Alexa Fluor 488 or 594 secondary antibodies (Molecular probes) for 1 hr, cover glasses were mounted using Vectashield mounting medium (Vector) and analyzed using a confocal fluorescence microscope (FV-300, Olympus).

### Immunoprecipitation

Cells were washed with PBS and then treated with 20 mM DSP (Pierce) in PBS for 30 min at RT. After washing with PBS, cells were solubilized in TNE buffer (150 mM Tris-Hcl (pH 7.5), 500 mM NaCl, 1 mM EDTA, 1% TritonX-100, 0.1% SDS). The lysates were incubated with the indicated antibodies and Protein-G Sepharose for 2hr at 4C. The beads were washed five times with TNE buffer and proteins were eluted by boiling in SDS-PAGE sampling buffer.

### GST pulldown assay

Recombinant proteins were expressed in BL21 bacterial strains and purified using GSH-Sepharose (Amersham) or Amylose Resin (New England Biolabs). ERp44 was removed from GST-ERp44 by digestion with Thrombin Protease (Amersham Biosciences). For Figure 2B, GST or GST fusion proteins were incubated with MBP, or MBP fusion proteins for 1 hr at 4C in acidic solution (30 mM NaOAc (pH 5.2), 150 mM NaCl, 4mM CaCl2, 0. 1 % Triton X-100). For Figure 2C and 2, proteins were incubated in neutral solution (20 mM Tris-HCl (pH 7.5), 150 mM NaCl, 5 mM EGTA, 0.1% Triton X-100)with or without 3 mM DTT For Figure 2E, GST-1L3V and MBP-ERp44 were incubated in pH 7.5 buffer or pH 7.5 buffer, in which EGTA was replaced to indicated concentration of CaCl2. Experiments in Figure 2H and 5E were carried out in neutral solution containing 3 mM DTT. Each mixture was then incubated for additional 2 hr with GSH-Sepharose. The beads were washed five times and subjected to western blot analysis.

### RNA interference for ERp44

SiRNA duplexes were purchased from Dharmacon. Target sequences were:

siContro1-C (5'-AAGUAGUGUAUGCUAGAGUGG-3' ) siERp44-C (5'-

AAGUAGUGUUUGCCAGAGUUG-3 '), siContro1-3U (5'-
AACAGCAGCAUCAACCUACGU-3 '), siERp44-3U (5'-
AACAGCAGCAUCAACCUACGU-3').

### Ca²⁺ imaging

After 24-48 hours following transfection, HeLa, COS-7, DT40 - KMN60, DT40-1KO,and DT40 - TKO cells were incubated for 30 min with 5 µM fura-2 acetoxymethyl ester (Dojindo). The fura-2-loaded cells were placed on the stage of an inverted fluorescence microscope (IX-70; Olympus) and perfused with balanced salt solution (BSS). Image capture and processing were perfomed with Argus 50/CA system (Hamamatsu photonics) at RT. With alternate illumination at 340 nm and 380 nm excitation, pairs of fluorescence images (F340 and F380 respectively) were obtained through an objectivelens (UApo 20/340 or UplanApo 100 Oil Iris; Olympus) and an emission filter (510-550 nm), captured with a silicon-intensified-target video camera (C2400-8;Hamamatsu Photonics). Fluorescence images of GFP and RFP were separately acquired and saved in the computer in the same optical fields as those for Ca²⁺ imaging.

### Planar Lipid Bilayer Experiments

Single-channel recordings of IP₃R1 in mouse cerebellar microsomes were performed as previously described (Michikawa et al., 1999) at 20 mV transmembrane potential using 53 mM Ba²⁺. The cis chamber contained 108 mM Tris dissolved in 250 mM HEPES (pH 7.33), 1.11 mM K2-H-HEDTA (N-hydroxymethylethylenediamine.N,N',N'-triaceticacid), 0.12 mM K-Ca²⁺-HEDTA. The trans chamber contained 250 mM HEPES (pH7.33), 53 mM Ba (OH)2, 3 mM DTT IP₃R1 were activated by addition of 6.4 mM IP₃ and 0.5 mM ATP to the cis chamber. Purified MBP or MBP-ERp44 was added directly to transside. The IP₃R1 single channel currents were amplified (Axon Instruments),filtered at 1 kHz by a low-pass Bessel filter (model, NF Instruments), sampled at 10kHz, and analyzed (Michikawa et al., 1999). Single-channel amplitudes were determined by fitting Gaussian curves to the all-point amplitude histograms of the datafiltered at 1 kHz. For presentation of the current traces, data were filtered at 0.5 kHz.

[Sequence List]
<1 1 0>Head of the University of Tokyo
<1 2 0>
<1 3 0>U2003P425
<1 6 0>4
<2 1 0>1
<2 1 1>4 0 6
<2 1 2>amino acid
<2 1 3>mouse
<4 0 0>1

Met Asn Pro Ala Val Phe Leu Ser Leu Ala Asp Leu Arg Cys Ser Leu
1               5                   10                  15

Leu Leu Leu Val Thr Ser Ile Phe Thr Pro Ile Thr Ala Glu Ile Ala
            20                  25                  30

Ser Leu Asp Ser Glu Asn Ile Asp Glu Ile Leu Asn Asn Ala Asp Val
        35                  40                  45

Ala Leu Val Asn Phe Tyr Ala Asp Trp Cys Arg Phe Ser Gln Met Leu
    50                  55                  60

His Pro Ile Phe Glu Glu Ala Ser Asp Val Ile Lys Glu Glu Tyr Pro
65                  70                  75                  80

Asp Lys Asn Gln Val Val Phe Ala Arg Val Asp Cys Asp Gln His Ser
                85                  90                  95

Asp Ile Ala Gln Arg Tyr Arg Ile Ser Lys Tyr Pro Thr Leu Lys Leu
            100                 105                 110

Phe Arg Asn Gly Met Met Met Lys Arg Glu Tyr Arg Gly Gln Arg Ser
        115                 120                 125

Val Lys Ala Leu Ala Asp Tyr Ile Arg Gln Gln Lys Ser Asn Pro Val
    130                 135                 140

His Glu Ile Gln Ser Leu Asp Glu Val Thr Asn Leu Asp Arg Ser Lys
145                 150                 155                 160

Arg Asn Ile Ile Gly Tyr Phe Glu Gln Lys Asp Ser Glu Asn Tyr Arg
                165                 170                 175

Val Phe Glu Arg Val Ala Ser Ile Leu His Asp Asp Cys Ala Phe Leu
            180                 185                 190

Ser Ala Phe Gly Asp Leu Ser Lys Pro Glu Arg Tyr Asn Gly Asp Asn
        195                 200                 205

Val Ile Tyr Lys Pro Pro Gly Arg Ser Ala Pro Asp Met Val Tyr Leu
    210                 215                 220

Gly Ser Met Thr Asn Phe Asp Val Thr Tyr Asn Trp Ile Gln Asp Lys
225                 230                 235                 240

Cys Val Pro Leu Val Arg Glu Ile Thr Phe Glu Asn Gly Glu Glu Leu
                245                 250                 255

Thr Glu Glu Gly Leu Pro Phe Leu Ile Leu Phe His Met Lys Asp Asp
            260                 265                 270

Thr Glu Ser Leu Glu Ile Phe Gln Asn Glu Val Ala Arg Gln Leu Ile
        275                 280                 285

Ser Glu Lys Gly Thr Ile Asn Phe Leu His Ala Asp Cys Asp Lys Phe
    290                 295                 300

Arg His Pro Leu Leu His Ile Gln Lys Thr Pro Ala Asp Cys Pro Val
305                 310                 315                 320

Ile Ala Ile Asp Ser Phe Arg His Met Tyr Val Phe Gly Asp Phe Lys
                325                 330                 335

Asp Val Leu Ile Pro Gly Lys Leu Lys Gln Phe Val Phe Asp Leu His
            340                 345                 350

Ser Gly Lys Leu His Arg Glu Phe His His Gly Pro Asp Pro Thr Asp
        355                 360                 365

Thr Ala Pro Gly Glu Gln Asp Gln Asp Val Ala Ser Ser Pro Pro Glu
    370                 375                 380

Ser Ser Phe Gln Lys Leu Ala Pro Ser Glu Tyr Arg Tyr Thr Leu Leu
385                 390                 395                 400

Arg Asp Arg Asp Glu Leu
                405

<210>2
<211>1221
<212>nucleic acid
<213>mouse
<400>2

atgaatcctg cggtcttcct gtctttagca gacctcagat gctccttgct gctcctggta 60
acttcaattt ttacacctat aacagctgaa atagcaagtc ttgattcaga gaatatagat 120
gaaattttaa ataatgctga tgttgcttta gtaaattttt atgctgactg gtgtcgtttc 180
agccagatgt tgcatccaat ttttgaggaa gcatctgatg tcattaagga agaatatcca 240
gataaaaatc aagtagtgtt tgccagagtt gattgtgatc agcactctga tatagcccag 300
aggtacagga taagcaaata cccaaccctg aaattatttc gtaatggaat gatgatgaag 360
agagaataca ggggccagcg atcagtgaaa gcgcttgcag actacatcag gcaacagaaa 420
agtaacccag tccacgagat tcagagtcta gatgaagtca ccaatcttga tcgcagtaag 480
agaaatatca ttggatactt tgagcagaag gattcagaaa attacagagt ttttgaaaga 540
gtagcaagta ttttgcatga tgactgtgcc ttcctttctg cttttggaga tctttcaaaa 600
ccagaaaggt acaacggaga caatgtaatc tacaaaccac ccgggcgttc tgcgccagac 660
atggtgtact tgggatctat gacaaatttt gatgtaactt acaactggat tcaagataaa 720
tgtgtccctc ttgtccgaga aataacattt gaaaatggag aggaattgac agaagaagga 780
ctgccttttc tcatactctt ccacatgaag gatgatacag aaagtttaga aatattccag 840
aatgaagtag cccggcagtt gataagtgaa aaaggtacaa taaatttctt acatgcagat 900
tgtgacaaat ttagacatcc tcttctccat atccagaaaa cgccagcaga ttgccctgtc 960
atagccattg acagttttag gcatatgtat gtttttggag actttaagga tgtattaatt 1020
cctggaaaac tcaagcagtt tgtatttgac ctacattctg gaaaattaca cagagaattc 1080
catcacggac ctgacccgac tgacacagcc ccaggagagc aagaccagga tgtagcgagc 1140
agtcctccag agagctcctt ccagaagctg gcgcccagcg agtataggta tactttattg 1200
agggatcgag atgagctgta a 1221

<210>3
<211>406
<212>amino acid
<213>human
<400>3

Met His Pro Ala Val Phe Leu Ser Leu Pro Asp Leu Arg Cys Ser Leu
1               5                   10                  15

Leu Leu Leu Val Thr Trp Val Phe Thr Pro Val Thr Thr Glu Ile Thr

            20                  25                  30

Ser Leu Asp Thr Glu Asn Ile Asp Glu Ile Leu Asn Asn Ala Asp Val
        35                  40                  45

Ala Leu Val Asn Phe Tyr Ala Asp Trp Cys Arg Phe Ser Gln Met Leu
    50                  55                  60

His Pro Ile Phe Glu Glu Ala Ser Asp Val Ile Lys Glu Glu Phe Pro
65                  70                  75                  80

Asn Glu Asn Gln Val Val Phe Ala Arg Val Asp Cys Asp Gln His Ser
                85                  90                  95

Asp Ile Ala Gln Arg Tyr Arg Ile Ser Lys Tyr Pro Thr Leu Lys Leu
            100                 105                 110

Phe Arg Asn Gly Met Met Met Lys Arg Glu Tyr Arg Gly Gln Arg Ser
        115                 120                 125

Val Lys Ala Leu Ala Asp Tyr Ile Arg Gln Gln Lys Ser Asp Pro Ile
    130                 135                 140

Gln Glu Ile Arg Asp Leu Ala Glu Ile Thr Thr Leu Asp Arg Ser Lys
145                 150                 155                 160

Arg Asn Ile Ile Gly Tyr Phe Glu Gln Lys Asp Ser Asp Asn Tyr Arg
                165                 170                 175

Val Phe Glu Arg Val Ala Asn Ile Leu His Asp Asp Cys Ala Phe Leu
            180                 185                 190

Ser Ala Phe Gly Asp Val Ser Lys Pro Glu Arg Tyr Ser Gly Asp Asn
        195                 200                 205

Ile Ile Tyr Lys Pro Pro Gly His Ser Ala Pro Asp Met Val Tyr Leu
    210                 215                 220

Gly Ala Met Thr Asn Phe Asp Val Thr Tyr Asn Trp Ile Gln Asp Lys
225                 230                 235                 240

Cys Val Pro Leu Val Arg Glu Ile Thr Phe Glu Asn Gly Glu Glu Leu
                245                 250                 255

Thr Glu Glu Gly Leu Pro Phe Leu Ile Leu Phe His Met Lys Glu Asp
            260                 265                 270

Thr Glu Ser Leu Glu Ile Phe Gln Asn Glu Val Ala Arg Gln Leu Ile
        275                 280                 285

Ser Glu Lys Gly Thr Ile Asn Phe Leu His Ala Asp Cys Asp Lys Phe
    290                 295                 300

Arg His Pro Leu Leu His Ile Gln Lys Thr Pro Ala Asp Cys Pro Val
305                 310                 315                 320

Ile Ala Ile Asp Ser Phe Arg His Met Tyr Val Phe Gly Asp Phe Lys
                325                 330                 335

Asp Val Leu Ile Pro Gly Lys Leu Lys Gln Phe Val Phe Asp Leu His
            340                 345                 350

Ser Gly Lys Leu His Arg Glu Phe His His Gly Pro Asp Pro Thr Asp
        355                 360                 365

Thr Ala Pro Gly Glu Gln Ala Gln Asp Val Ala Ser Ser Pro Pro Glu
    370                 375                 380

Ser Ser Phe Gln Lys Leu Ala Pro Ser Glu Tyr Arg Tyr Thr Leu Leu
385                 390                 395                 400

Arg Asp Arg Asp Glu Leu
                405

<210>4
<211>1221
<212>nucleic acid
<213>human
<400>4
atgcatcctg ccgtcttcct atccttaccc gacctcagat gctcccttct gctcctggta 60
acttgggttt ttactcctgt aacaactgaa ataacaagtc ttgatacaga gaatatagat 120
gaaattttaa acaatgctga tgttgcttta gtaaattttt atgctgactg gtgtcgtttc 180
agtcagatgt tgcatccaat ttttgaggaa gcttccgatg tcattaagga agaatttcca 240
aatgaaaatc aagtagtgtt tgccagagtt gattgtgatc agcactctga catagcccag 300
agatacagga taagcaaata cccaaccctc aaattgtttc gtaatgggat gatgatgaag 360
agagaataca ggggtcagcg atcagtgaaa gcattggcag attacatcag gcaacaaaaa 420
agtgacccca ttcaagaaat tcgggactta gcagaaatca ccactcttga tcgcagcaaa 480
agaaatatca ttggatattt tgagcaaaag gactcggaca actatagagt ttttgaacga 540
gtagcgaata ttttgcatga tgactgtgcc tttctttctg catttgggga tgtttcaaaa 600
ccggaaagat atagtggcga caacataatc tacaaaccac cagggcattc tgctccggat 660
atggtgtact tgggagctat gacaaatttt gatgtgactt acaattggat tcaagataaa 720
tgtgttcctc ttgtccgaga aataacattt gaaaatggag aggaattgac agaagaagga 780

ctgccttttc tcatactctt tcacatgaaa gaagatacag aaagtttaga aatattccag 840
aatgaagtag ctcggcaatt aataagtgaa aaaggtacaa taaacttttt acatgccgat 900
tgtgacaaat ttagacatcc tcttctgcac atacagaaaa ctccagcaga ttgtcctgta 960
atcgctattg acagctttag gcatatgtat gtgtttggag acttcaaaga tgtattaatt 1020
cctggaaaac tcaagcaatt cgtatttgac ttacattctg gaaaactgca cagagaattc 1080
catcatggac ctgacccaac tgatacagcc ccaggagagc aagcccaaga tgtagcaagc 1140
agtccacctg agagctcctt ccagaaacta gcacccagtg aatataggta tactctattg 1200
agggatcgag atgagcttta a 1221

[Sequence List]
<110>Head of the University of Tokyo
<120>
<130>U2003P425
<160>4
<210>1
<211>406
<212>amino acid
<213>mouse
<400>1

Met Asn Pro Ala Val Phe Leu Ser Leu Ala Asp Leu Arg Cys Ser Leu
1               5                   10                  15

Leu Leu Leu Val Thr Ser Ile Phe Thr Pro Ile Thr Ala Glu Ile Ala
            20                  25                  30

Ser Leu Asp Ser Glu Asn Ile Asp Glu Ile Leu Asn Asn Ala Asp Val
        35                  40                  45

Ala Leu Val Asn Phe Tyr Ala Asp Trp Cys Arg Phe Ser Gln Met Leu
    50                  55                  60

His Pro Ile Phe Glu Glu Ala Ser Asp Val Ile Lys Glu Glu Tyr Pro
65                  70                  75                  80

Asp Lys Asn Gln Val Val Phe Ala Arg Val Asp Cys Asp Gln His Ser
                85                  90                  95

Asp Ile Ala Gln Arg Tyr Arg Ile Ser Lys Tyr Pro Thr Leu Lys Leu
            100                 105                 110

Phe Arg Asn Gly Met Met Met Lys Arg Glu Tyr Arg Gly Gln Arg Ser
        115                 120                 125

Val Lys Ala Leu Ala Asp Tyr Ile Arg Gln Gln Lys Ser Asn Pro Val
    130                 135                 140

His Glu Ile Gln Ser Leu Asp Glu Val Thr Asn Leu Asp Arg Ser Lys
145                 150                 155                 160

Arg Asn Ile Ile Gly Tyr Phe Glu Gln Lys Asp Ser Glu Asn Tyr Arg
                165                 170                 175

Val Phe Glu Arg Val Ala Ser Ile Leu His Asp Asp Cys Ala Phe Leu
            180                 185                 190

Ser Ala Phe Gly Asp Leu Ser Lys Pro Glu Arg Tyr Asn Gly Asp Asn
        195                 200                 205

Val Ile Tyr Lys Pro Pro Gly Arg Ser Ala Pro Asp Met Val Tyr Leu
    210                 215                 220

Gly Ser Met Thr Asn Phe Asp Val Thr Tyr Asn Trp Ile Gln Asp Lys
225                 230                 235                 240

Cys Val Pro Leu Val Arg Glu Ile Thr Phe Glu Asn Gly Glu Glu Leu
                245                 250                 255

Thr Glu Glu Gly Leu Pro Phe Leu Ile Leu Phe His Met Lys Asp Asp
            260                 265                 270

Thr Glu Ser Leu Glu Ile Phe Gln Asn Glu Val Ala Arg Gln Leu Ile
        275                 280                 285

Ser Glu Lys Gly Thr Ile Asn Phe Leu His Ala Asp Cys Asp Lys Phe
    290                 295                 300

Arg His Pro Leu Leu His Ile Gln Lys Thr Pro Ala Asp Cys Pro Val
305                 310                 315                 320

Ile Ala Ile Asp Ser Phe Arg His Met Tyr Val Phe Gly Asp Phe Lys
                325                 330                 335

Asp Val Leu Ile Pro Gly Lys Leu Lys Gln Phe Val Phe Asp Leu His
            340                 345                 350

Ser Gly Lys Leu His Arg Glu Phe His His Gly Pro Asp Pro Thr Asp
        355                 360                 365

Thr Ala Pro Gly Glu Gln Asp Gln Asp Val Ala Ser Ser Pro Pro Glu
    370                 375                 380

Ser Ser Phe Gln Lys Leu Ala Pro Ser Glu Tyr Arg Tyr Thr Leu Leu
385                 390                 395                 400

Arg Asp Arg Asp Glu Leu
                405

<210>2
<211>1221
<212>nucleic acid
<213>mouse
<400>2

atgaatcctg cggtcttcct gtctttagca gacctcagat gctccttgct gctcctggta 60
acttcaattt ttacacctat aacagctgaa atagcaagtc ttgattcaga gaatatagat 120
gaaattttaa ataatgctga tgttgcttta gtaaattttt atgctgactg gtgtcgtttc 180
agccagatgt tgcatccaat ttttgaggaa gcatctgatg tcattaagga agaatatcca 240
gataaaaatc aagtagtgtt tgccagagtt gattgtgatc agcactctga tatagcccag 300
aggtacagga taagcaaata cccaaccctg aaattatttc gtaatggaat gatgatgaag 360
agagaataca ggggccagcg atcagtgaaa gcgcttgcag actacatcag gcaacagaaa 420
agtaacccag tccacgagat tcagagtcta gatgaagtca ccaatcttga tcgcagtaag 480
agaaatatca ttggatactt tgagcagaag gattcagaaa attacagagt ttttgaaaga 540
gtagcaagta ttttgcatga tgactgtgcc ttcctttctg cttttggaga tctttcaaaa 600
ccagaaaggt acaacggaga caatgtaatc tacaaaccac ccgggcgttc tgcgccagac 660
atggtgtact tgggatctat gacaaatttt gatgtaactt acaactggat tcaagataaa 720
tgtgtccctc ttgtccgaga aataacattt gaaaatggag aggaattgac agaagaagga 780
ctgccttttc tcatactctt ccacatgaag gatgatacag aaagtttaga aatattccag 840
aatgaagtag cccggcagtt gataagtgaa aaaggtacaa taaatttctt acatgcagat 900
tgtgacaaat ttagacatcc tcttctccat atccagaaaa cgccagcaga ttgccctgtc 960
atagccattg acagttttag gcatatgtat gtttttggag actttaagga tgtattaatt 1020
cctggaaaac tcaagcagtt tgtatttgac ctacattctg gaaaattaca cagagaattc 1080
catcacggac ctgacccgac tgacacagcc ccaggagagc aagaccagga tgtagcgagc 1140
agtcctccag agagctcctt ccagaagctg gcgcccagcg agtataggta tactttattg 1200
agggatcgag atgagctgta a 1221

<210>3
<211>406
<212>amino acid
<213>human
<400>3

Met His Pro Ala Val Phe Leu Ser Leu Pro Asp Leu Arg Cys Ser Leu
1               5                   10                  15

Leu Leu Leu Val Thr Trp Val Phe Thr Pro Val Thr Thr Glu Ile Thr

            20                  25                  30

Ser Leu Asp Thr Glu Asn Ile Asp Glu Ile Leu Asn Asn Ala Asp Val
        35                  40                  45

Ala Leu Val Asn Phe Tyr Ala Asp Trp Cys Arg Phe Ser Gln Met Leu
    50                  55                  60

His Pro Ile Phe Glu Glu Ala Ser Asp Val Ile Lys Glu Glu Phe Pro
65                  70                  75                  80

Asn Glu Asn Gln Val Val Phe Ala Arg Val Asp Cys Asp Gln His Ser
                85                  90                  95

Asp Ile Ala Gln Arg Tyr Arg Ile Ser Lys Tyr Pro Thr Leu Lys Leu
            100                 105                 110

Phe Arg Asn Gly Met Met Met Lys Arg Glu Tyr Arg Gly Gln Arg Ser
        115                 120                 125

Val Lys Ala Leu Ala Asp Tyr Ile Arg Gln Gln Lys Ser Asp Pro Ile
    130                 135                 140

Gln Glu Ile Arg Asp Leu Ala Glu Ile Thr Thr Leu Asp Arg Ser Lys
145                 150                 155                 160

Arg Asn Ile Ile Gly Tyr Phe Glu Gln Lys Asp Ser Asp Asn Tyr Arg
                165                 170                 175

Val Phe Glu Arg Val Ala Asn Ile Leu His Asp Asp Cys Ala Phe Leu
            180                 185                 190

Ser Ala Phe Gly Asp Val Ser Lys Pro Glu Arg Tyr Ser Gly Asp Asn
        195                 200                 205

Ile Ile Tyr Lys Pro Pro Gly His Ser Ala Pro Asp Met Val Tyr Leu
    210                 215                 220

Gly Ala Met Thr Asn Phe Asp Val Thr Tyr Asn Trp Ile Gln Asp Lys
225                 230                 235                 240

Cys Val Pro Leu Val Arg Glu Ile Thr Phe Glu Asn Gly Glu Glu Leu
                245                 250                 255

Thr Glu Glu Gly Leu Pro Phe Leu Ile Leu Phe His Met Lys Glu Asp
            260                 265                 270

Thr Glu Ser Leu Glu Ile Phe Gln Asn Glu Val Ala Arg Gln Leu Ile
        275                 280                 285

Ser Glu Lys Gly Thr Ile Asn Phe Leu His Ala Asp Cys Asp Lys Phe
    290                 295                 300

Arg His Pro Leu Leu His Ile Gln Lys Thr Pro Ala Asp Cys Pro Val
305                 310                 315                 320

Ile Ala Ile Asp Ser Phe Arg His Met Tyr Val Phe Gly Asp Phe Lys
                325                 330                 335

Asp Val Leu Ile Pro Gly Lys Leu Lys Gln Phe Val Phe Asp Leu His
            340                 345                 350

Ser Gly Lys Leu His Arg Glu Phe His His Gly Pro Asp Pro Thr Asp
        355                 360                 365

Thr Ala Pro Gly Glu Gln Ala Gln Asp Val Ala Ser Ser Pro Pro Glu
    370                 375                 380

Ser Ser Phe Gln Lys Leu Ala Pro Ser Glu Tyr Arg Tyr Thr Leu Leu
385                 390                 395                 400

Arg Asp Arg Asp Glu Leu
                405

<210>4
<211>1221
<212>nucleic acid
<213>human
<400>4
atgcatcctg ccgtcttcct atccttaccc gacctcagat gctcccttct gctcctggta 60
acttgggttt ttactcctgt aacaactgaa ataacaagtc ttgatacaga gaatatagat 120
gaaattttaa acaatgctga tgttgcttta gtaaattttt atgctgactg gtgtcgtttc 180
agtcagatgt tgcatccaat ttttgaggaa gcttccgatg tcattaagga agaatttcca 240
aatgaaaatc aagtagtgtt tgccagagtt gattgtgatc agcactctga catagcccag 300
agatacagga taagcaaata cccaaccctc aaattgtttc gtaatgggat gatgatgaag 360
agagaataca ggggtcagcg atcagtgaaa gcattggcag attacatcag gcaacaaaaa 420
agtgacccca ttcaagaaat tcgggactta gcagaaatca ccactcttga tcgcagcaaa 480
agaaatatca ttggatattt tgagcaaaag gactcggaca actatagagt ttttgaacga 540
gtagcgaata ttttgcatga tgactgtgcc tttctttctg catttgggga tgtttcaaaa 600
ccggaaagat atagtggcga caacataatc tacaaaccac cagggcattc tgctccggat 660
atggtgtact tgggagctat gacaaatttt gatgtgactt acaattggat tcaagataaa 720
tgtgttcctc ttgtccgaga aataacattt gaaaatggag aggaattgac agaagaagga 780

ctgccttttc tcatactctt tcacatgaaa gaagatacag aaagtttaga aatattccag 840

aatgaagtag ctcggcaatt aataagtgaa aaaggtacaa taaacttttt acatgccgat 900

tgtgacaaat ttagacatcc tcttctgcac atacagaaaa ctccagcaga ttgtcctgta 960

atcgctattg acagctttag gcatatgtat gtgtttggag acttcaaaga tgtattaatt 1020

cctggaaaac tcaagcaatt cgtatttgac ttacattctg gaaaactgca cagagaattc 1080

catcatggac ctgacccaac tgatacagcc ccaggagagc aagcccaaga tgtagcaagc 1140

agtccacctg agagctcctt ccagaaacta gcacccagtg aatataggta tactctattg 1200

agggatcgag atgagcttta a 1221

## Claims

1. A molecular of controlling a calcium comprising the following (a) or (b):
(a) an amino acid sequence shown in an amino acid sequence number 1-406 of a sequence No.1 of a sequence table, or
(b) an amino acid sequence wherein a part of the amino acid sequence of the amino acid sequence number 1-406 is deleted, substituted or added.

2. A recombinant vector comprising the following (a) or (b):
(a) a nucleic acid comprising a base sequence shown in a base sequence number 1-1221 of a sequence No.2 of a sequence table, or
(b) a nucleic acid wherein a part of the base sequence of the base sequence number 1-1221 is deleted, substituted or added, and the nucleic acid has a homology of 80% for the base sequence.

3. A molecular of controlling a calcium comprising the following (a) or (b):
(a) an amino acid sequence shown in an amino acid sequence number 1-406 of a sequence No.3 of a sequence table, or
(b) an amino acid sequence wherein a part of the amino acid sequence of the amino acid sequence number 1-406 is deleted, substituted or added.

4. A recombinant vector comprising the following (a) or (b):
(a) a nucleic acid comprising a base sequence shown in a base sequence number 1-1221 of a sequence No.4 of a sequence table, or
(b) a nucleic acid wherein a part of the base sequence of the base sequence number 1-1221 is deleted, substituted or added, and the nucleic acid has a homology of 80% for the base sequence.

5. An antibody against the polypeptide described in claims 1 or 2.

6. A cultured cell **characterized in that** the cell is transfected by said recombinant vector described in claims 2-4.

7. The cultured cell is **characterized in that** an ability of Ca²⁺ release is reduced by over-expressing the polypeptide described in claims 1 or 3.

8. The cultured cell is **characterized in that** an ability of Ca²⁺ release is increased by restraining the express of the polypeptide described in claims 1 or 3.

9. The cultured cell is **characterized** that an ability of Ca²⁺ release is increased by introducing the antibody described in claim 5 into said cultured cell and inducing an antigen-antibody reaction between the antibody and the polypeptide of claims 1 or 3.
